(19) Europäisches Patentamt / European Patent Office / Office européen des brevets

(11) **EP 2 890 978 B1**

(12) **EUROPEAN PATENT SPECIFICATION**

(45) Date of publication and mention
of the grant of the patent:
**27.09.2017 Bulletin 2017/39**

(21) Application number: **13756102.3**

(22) Date of filing: **28.08.2013**

(51) Int Cl.:
**G01N 33/18** $^{(2006.01)}$     **C02F 1/00** $^{(2006.01)}$

(86) International application number:
**PCT/EP2013/067759**

(87) International publication number:
**WO 2014/033145 (06.03.2014 Gazette 2014/10)**

(54) **PROCESSING DATA OBTAINED BY OPERATING A LIQUID TREATMENT SYSTEM**

VERARBEITUNG VON DATEN EINES FLÜSSIGKEITSBEHANDLUNGSSYSTEMS

TRAITEMENT DES DONNÉES D'UN SYSTÈME DE TRAITEMENT DE LIQUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.08.2012 EP 12182117**

(43) Date of publication of application:
**08.07.2015 Bulletin 2015/28**

(73) Proprietor: **Brita GmbH**
**65232 Taunusstein (DE)**

(72) Inventors:
• **WEIDNER, Peter**
**92444 Rötz (DE)**

• **CONRADT, Berthold**
**65205 Wiesbaden (DE)**
• **NAGEL, Thomas**
**35796 Weinbach (DE)**

(74) Representative: **van Lookeren Campagne,**
**Constantijn August**
**Meissner Bolte Patentanwälte**
**Rechtsanwälte Partnerschaft mbB**
**Bankgasse 3**
**90402 Nürnberg (DE)**

(56) References cited:
**EP-A1- 2 228 129**     **DE-A1-102005 005 039**
**DE-A1-102009 047 254**

**Description**

The invention relates to a method of processing data obtained by operating a liquid treatment system including a liquid treatment part containing at least one liquid treatment medium, which method includes:

receiving a measurement signal, values of which are representative of a first parameter, the first parameter being a parameter of the liquid depending partially on a concentration of at least one component removable to at least some extent by the liquid treatment part from liquid flowing through the liquid treatment part and also on that of other components that are not removable,
wherein the signal is a signal originating from a sensor located downstream of the liquid treatment part;
determining values of the measure of the concentration of components removable by the liquid treatment part on the basis of at least respective values of the measurement signal; and
determining values of at least one second parameter, each second parameter corresponding to an integral of a variable from a point in time at which liquid treatment medium in the liquid treatment part is in an initial state over periods during which liquid flows through the liquid treatment part, wherein the variable is at least dependent on one of a rate at which liquid flows through the liquid treatment part during use and a rate at which liquid flows through the liquid treatment system during use.

The invention also relates to a system for processing data obtained by operating a liquid treatment system including a liquid treatment part containing at least one liquid treatment medium,
wherein the system includes an interface for receiving a measurement signal, values of which are representative of a first parameter, the first parameter being a parameter of the liquid depending partly on a concentration of at least one component removable to at least some extent by the liquid treatment part from liquid flowing through the liquid treatment part and also on that of other components that are not removable,
wherein the system is configured to process a measurement signal originating from a sensor located downstream of the liquid treatment part,
wherein the system is configured to determine values of the measure of the concentration of components removable to at least some extent by the liquid treatment part from liquid flowing through the liquid treatment part, and
wherein the system is configured to determine values of at least one second parameter, each second parameter corresponding to an integral of a variable from a point in time at which liquid treatment medium in the liquid treatment part is in an initial state over periods during which liquid flows through the liquid treatment part, wherein the variable is at least dependent on one of a rate at which liquid flows through the liquid treatment part during use and a rate at which liquid flows through the liquid treatment system during use.

The invention also relates to a liquid treatment system.

The invention also relates to a computer program.

DE 10 2005 005 039 A1 discloses a water-conducting household appliance with a device for capturing the hardness of a liquid and a signaling device controlled by regulating and/or control electronics for outputting information relating to the hardness. The signaling device serves to output information on a hardness level that is impermissibly above or below a threshold value.

EP 2 228 129 A1 discloses a method of monitoring a water softening apparatus, wherein the water softening apparatus comprises a softening device, in particular comprising at least one tank with ion exchange resin in it, a storage vessel, in which a solid regenerating salt for the production of a regenerating solution and/or a solution of regenerating agent is stored, a rinsing channel for draining off used regenerating solution and rinsing water during a regeneration and an electronic control device, in particular for automatic initiation and monitoring of a regeneration of the softening device through a solution of regenerating agent. The conductivity of the used solution of regenerating agent and/or of the rinsing water is determined during the regeneration by means of a conductivity sensor, and the conductivity determined experimentally is compared with a target conductivity profile stored in the electronic control device. If the target conductivity profile is stored as a function of the amount of liquid drained through the rinsing channel in the course of the regeneration, then certain variations in external conditions, in particular an inconstant pressure of the raw water, leave the comparability of the experimental conductivity values and the associated conductivity values of the target conductivity profile unaffected. In a preferred variant of the method, the control device monitors the hardness of the raw water flowing to the softening device as well as the amount of water softened by the softening device for determining a remaining capacity of the softening device and automatically initiates a regeneration of the softening device on time before exhaustion of the capacity of the softening device.

WO 2010/025697 A1 discloses a method of operating a water softening installation with an automatically adjustable blending device for mixing a blending water flow with a first, softened sub-flow and a second, untreated water-carrying sub-flow and with an electronic control device. The control device controls the adjustment position of the blending device with the aid of one or more experimentally determined, current measurements, so that the hardness of the water in the blended water flow is set to a given target value. The control device ignores at least one of the one or more measurements in controlling the adjustment position of the blending device under one or more defined operating conditions, instead taking the last valid measurement before the defined condition arose or a standard value stored in the electronic control device. In an embodiment, the defined operating conditions include times at which a hardness breakthrough of the softening installation exists. In another embodiment, the defined operating conditions include times at which less than a minimum amount of water has flowed through the water softening installation without interruption immediately prior to an intended evaluation of one or more of the current measurements. In a further embodiment, the water softening installation comprises a storage vat for providing regeneration solution and means for automatically performing a regeneration of the softening installation. The control device automatically triggers a regeneration of the softening installation in dependence on the withdrawal of softened water since the last regeneration. In a particular variant of this embodiment, the control device determines a remaining capacity of the softening installation in dependence on the withdrawals of softened water and the determined associated raw water hardness levels since the last regeneration that was triggered.

In the known method, values of the hardness are used only if they are within a certain, plausible range. The hardness values are based on measurements of the electrical conductivity of the water. Only if the rate of flow is so high that the softening installation cannot function or if water has only just started flowing is the use of a value of hardness of the water based on a current measurement suspended.

A problem of the known device is that, in the absence of one of a limited number of special situations, no account is taken of the fact that the hardness values themselves may be incorrect because the water softening installation may not conform to an ideal model underlying the calculation of the hardness on the basis of the electrical conductivity.

It is an object of the invention to provide a method, data processing system, liquid treatment system and computer program that use values representative of the parameter of the liquid downstream of the liquid treatment device and are able to take account of characteristics of actual liquid treatment devices commonly in use to reduce the likelihood that unreliable values of the measure of the concentration of components removable by the liquid treatment device are used.

This object is achieved according to one aspect by the method of processing data according to the invention, which is characterised by at least one of:

(i) determining whether to provide a value of the measure obtainable on the basis of at least a current value of the measurement signal as input for a further process; and
(ii) adapting the determination of values of the measure

in dependence on a current value of at least one of the second parameters.

Common liquid treatment devices including at least one liquid treatment medium remove, but often also give off, components that affect the first parameter. Because the method makes use of a signal originating from a sensor located downstream of the liquid treatment part, this signal will also capture influences of the liquid treatment media, rather than being purely based on the composition of the untreated liquid. On the other hand, a positive effect of the downstream measurement is that components removed by the liquid treatment medium do not influence the measurement as strongly as would be the case if all measurements were made upstream of the liquid treatment device. The present method is based on the recognition that influences of the liquid treatment medium or media in the liquid treatment part are quite strongly dependent on the phase of its useful life that the liquid treatment part is in. By determining values of a parameter corresponding to an integral of a variable representative of a rate of use of the liquid treatment part from a point in time at which the liquid treatment medium is in an initial state over periods during which liquid flows through the liquid treatment part, wherein the variable is dependent on at least a rate at which liquid flows through the liquid treatment part during use, the degree to which the liquid treatment part has been used already is quantified. This in turn may be used to determine whether to provide a current value of the measure obtainable on the basis of at least a current value of the measurement signal as input for a further process. In other words: the reliability of the current value of the measure is estimated on the basis of a determination of how far into its useful life the liquid treatment part is. Depending thereon, the step of determining a current value of the measure of the concentration of components removable by the liquid treatment part on the basis of at least a current value of the measurement signal is either not carried out at all, or it is carried out but the result not used. Alternatively or additionally, the step of determining a current value of the measure of the concentration of components removable by the liquid treatment part on the basis of at least a current value of the measurement signal is adapted in dependence on a current value of the parameter, so that the determination takes account of how far into its useful life the liquid treatment part currently is. Because the parameter used corresponds to an integral of a variable representative of a rate of use of the liquid treatment part from a point in time at which the liquid treatment medium is in an initial state over all subsequent periods during which liquid flows through the liquid treatment part, it is indeed a measure of the state of the liquid treatment part relative to its total useful lifetime.

It is observed that the further process may be a further process carried out by the same device as the one carrying out the method or a different device, the different device being configured to receive a signal representative of the values of the measure of the concentration of components removable by the liquid treatment medium from liquid flowing through the liquid treatment device. In the terminology used here, a current value corresponds to a latest available value.

In an embodiment of the method, the step of determining values of the measure of the concentration of components removable by the liquid treatment part includes determining a difference between a value of the first parameter at a first ratio of liquid treated by the liquid treatment part to liquid untreated by the liquid treatment part and a value of the first parameter at a different ratio of liquid treated by the liquid treatment part to liquid untreated by the liquid treatment part.

The ratios may have any value between and including zero and one. The ratios are known and, if not zero or one, used in the determination of the measure.

An ideal liquid treatment part will completely remove certain components from liquid flowing through it. From the difference between the values of the first parameter, and assuming the ratios are known, the change in the value of the first parameter due to the treatment by the liquid treatment part can be determined. A known relation between the first parameter and the measure of the concentration of removable components can then be used to separate out the contribution of the concentration of removable components to the first parameter value from that of other components that are not removable and on which the value of the first parameter also depends. However, such a determination will only yield a reliable value of the measure if the assumption that the liquid treatment part completely removes certain components from liquid flowing through it and leaves the concentration of other components on which the value of the first parameter depends unaffected is correct. In real-life situations, the performance of the liquid treatment part will vary. The removal may be incomplete or the liquid treatment part may add certain components that affect the value of the first parameter to the liquid. Taking account of the stage in its useful lifetime reached by the liquid treatment part enables the non-ideal behaviour of the liquid treatment part to be taken into account. This may be based on a model of the performance of the liquid treatment part over its useful life. It is thus possible to adapt the calculations used to arrive at a value of the measure on the basis of at least one value of the measurement signal. It is also possible to suspend the execution or use of the results of such calculations because the result would be too unreliable.

In a variant of this embodiment, wherein the liquid treatment system includes:

a first fluid path passing through the liquid treatment part;
a second fluid path bypassing the liquid treatment part, such that components removable by the liquid treatment part remain in liquid led through the second fluid path to at least a certain higher extent than in liquid led through the first fluid path;
a mixing location, where the first and second fluid paths join to mix liquid led through the first and second fluid paths; and
a device for adjusting a blending fraction corresponding to a proportion of liquid led through the second fluid path in liquid downstream of the mixing location,
the measurement signal is a signal originating from a sensor located downstream of the mixing location, and
the values of the first parameter at the first and second ratios are obtained by causing the device to adjust the blending fraction.

As a result, it is possible to obtain a value of the first parameter at a first ratio of liquid treated by the liquid treatment part to liquid untreated by the liquid treatment part and a value of the first parameter at a different ratio of liquid treated by the liquid treatment part to liquid untreated by the liquid treatment part without having to use both a sensor upstream of the liquid treatment part and a sensor downstream of the liquid treatment part. An effect is to make the method cheaper to implement. A further effect is that it is not necessary to calibrate sensors to take account of sensor drift. A sensor located upstream of a liquid treatment part would be exposed to liquid with a different composition than one located downstream of the liquid treatment part, so that one would expect a pair of such sensors to exhibit different levels of sensor drift. Moreover, the types of components removable by the liquid treatment part will typically be those that are detrimental to the longevity of liquid processing equipment, so that one might expect a sensor located upstream of the liquid treatment part also to have a relatively short useful life.

In a variant of this method, determining a value of the measure of the concentration of components removable by the liquid treatment part on the basis of values of the measurement signal includes:

causing the device to adjust the blending fraction from a first value to a second value;
obtaining the values of the first parameter at the first and second ratios; and
dividing the difference between values of the first parameter by a difference between the first and second values of the blending fraction.

The first and second values of the blending fraction may differ by only a small amount. Thus, an approximation of the (partial) derivative of the first parameter with respect to the blending fraction is obtained. This corresponds to the difference between the value of the first parameter immediately upstream of the liquid treatment part and the value of the first parameter immediately downstream of the liquid treatment part.

As an example, let the first parameter be represented as $s$, and let x represent the blending fraction. The value of the first parameter immediately upstream of the liquid treatment part can be represented as $s_0$ and the value immediately downstream of the liquid treatment part as $s_1$. Assuming complete removal of certain components by the liquid treatment part, the concentration of these certain components in the untreated liquid is a function of $\Delta s = s_0 - s_1$. The value of the first parameter $s(x)$ at a particular value x of the blending fraction equals:

$$s(x) = x \cdot s_0 + (1 - x) \cdot s_1 = (s_0 - s_1) \cdot x + s_1 = \Delta s \cdot x + s_1 \; . \tag{1}$$

The value $\Delta s$ can be obtained by taking the derivate $s'(x)$ of the first parameter with respect to the blending fraction $x$. For example, a small deviation of the blending fraction from a particular set value $x^*$ can be used to approximate the derivative as follows:

$$s'(x^*) = \frac{s\left(x^* + \Delta x / 2\right) - s\left(x^* - \Delta x / 2\right)}{\Delta x} \; . \tag{2}$$

Using only small deviations has the effect that the concentration of removable components in the liquid downstream of the mixing location does not change significantly. It can thus remain at an optimum for appliances arranged to process the liquid provided downstream of the mixing location. It is not necessary to direct the mix of treated and untreated liquid down the drain because the concentration of components removable by the liquid treatment part in this mix is unsuitable for the appliance.

Indeed, in an embodiment of the method, wherein the liquid treatment system includes:

a first fluid path passing through the liquid treatment part;
a second fluid path bypassing the liquid treatment part, such that at least one component removable by the liquid treatment part remains in liquid led through the second fluid path to at least a certain higher extent than in liquid led through the first fluid path;
a mixing location, where the first and second fluid paths join to mix liquid led through the first and second fluid paths; and
a device for adjusting a blending fraction corresponding to a proportion of liquid led through the second fluid path in liquid downstream of the mixing location, the further process includes causing the device to adjust the blending fraction.

Thus, in this embodiment, the blending fraction is regulated or controlled. This is done in dependence on the measure of the concentration in untreated liquid of components removable from the liquid by the liquid treatment part, but only if the measure as determined on the basis of values of the measurement signal is reliable. Otherwise, control can be effected using a different input variable or the blending fraction can be set in accordance with one or more stored values or settings. The method can operate with a liquid treatment part that is based on the use of a liquid treatment medium or media that remove certain components to a slowly varying or essentially constant extent, and still allow for adjustment of the concentration of those components by mixing untreated and treated liquid. As an example, it is possible to provide water with a desired level of hardness or pH, without having to use an electrophysical water treatment device.

An embodiment of the method, wherein the liquid treatment system includes a replaceable cartridge including the liquid treatment part, further includes

detecting a replacement of the cartridge and

in response to detecting the replacement of the cartridge, setting an initial value of the second parameter.

This embodiment has the effect that the liquid treatment part can include a liquid treatment medium or media that is exhausted over a certain period of use and cannot then be easily regenerated on site. Rather, the cartridge is replaced and the cartridge containing the exhausted medium is returned to the supplier for regeneration of the exhausted medium. For the method to track the progress of the liquid treatment part, it is expedient to detect a change of cartridge automatically, so that the value of the second parameter can be reset.

A further embodiment of the method, wherein the liquid treatment system includes a replaceable cartridge including the liquid treatment part, further includes

obtaining data identifying a type of the cartridge from among a plurality of types, and

carrying out at least one of the determination of whether to provide a value of the measure obtainable on the basis of at least a current value of the measurement signal as input for a further process and the adaptation of the determination of values of the measure in dependence on the current value of at least one of the second parameters and the identified type.

This embodiment allows the method to function with cartridges of different capacity, for example. Different threshold values of the second parameter may be stored in association with different ones of the plurality of types. The embodiment can also cope with cartridges containing different media or mixes of media. In one variant, at least one of the determination of whether to provide a current value of the measure obtainable on the basis of at least a current value of the measurement signal as input for a further process and the adaptation of the determination of values of the measure in dependence on the current value of the second parameter and the identified type is carried out only if the type is one of a certain sub-set of possible types. Thus, where the cartridge is not in the sub-set, the measurement signal is never used. As an example, this feature can be used to distinguish between water treatment cartridges arranged to soften water and those also arranged to remove gypsum (calcium sulphate dihydrate), with the use of the measurement signal to determine the temporary hardness of untreated water being suspended when the cartridge is of the latter type.

In an embodiment, wherein the liquid treatment system includes a replaceable cartridge including the liquid treatment part, the method further includes at least one of reading and writing data from or to a tag attached to the replaceable cartridge.

In a variant, the data is transferred wirelessly.

In a variant, data written to the tag includes a current value of at least one of the second parameters.

An effect is that the liquid treatment cartridge can be removed and re-inserted into the same or a different liquid treatment system. The current value representative of the stage in its useful life that it has reached can then be read from the tag.

Consequently, in a further variant, an initial value of at least one of the second parameters is read from the tag upon placement of the liquid treatment cartridge in the liquid treatment system.

Determining values of this second parameter thus involves adding to the initial value a value corresponding to an integral of a variable representative of a rate of use of at least part of the liquid treatment system from the moment of insertion of the liquid treatment cartridge into the liquid treatment system over periods during which liquid flows through the liquid treatment part. The method therefore functions even if a partially used liquid treatment cartridge is inserted into the liquid treatment system.

In a variant, data read from the tag includes data representative of at least one threshold value of at least one of the second parameters, and the threshold value is used in at least one of

(i) determining whether to provide a current value of the measure obtainable on the basis of at least a current value of the measurement signal as input for a further process; and
(ii) adapting the determination of values of the measure

in dependence on a current value of at least one of the second parameters.

Thus, the system carrying out the method can deal with liquid treatment cartridges with different capacities. Data representative of at least one threshold value of at least one of the second parameters may be data suitable for use as a key into a database in which the threshold values are included. Alternatively, the data may allow the threshold values to be determined independently of any further data, in which case the device executing the method need not be programmed with all possible threshold values or updated when a new type of liquid treatment cartridge becomes available for use. It can also deal with changes in the composition of the liquid treatment medium or media without requiring an update.

In an embodiment, the values of the measurement signal are representative of one of:

electrical conductivity; and
electrical conductivity adjusted for deviation of a temperature of the liquid from a reference temperature value.

The electrical conductivity of a liquid is a suitable variable for the method, because it depends on the concentration of dissolved solids in the liquid, in particular also on the ion concentration. The concentration of a certain sub-set of all ion species has an influence on properties of the liquid that commonly need to be controlled, such as hardness (temporary or permanent) and pH. The electrical conductivity is easy to measure. If, additionally, the step of determining values of the measure of the concentration of components removable by the liquid treatment part includes determining a difference between a value of the first parameter at a first ratio of liquid treated by the liquid treatment part to liquid untreated by the liquid treatment part and a value of the first parameter at a different ratio of liquid treated by the liquid treatment part to liquid untreated by the liquid treatment part, then the use of ion-selective conductivity sensors is not required.

If the values of the measurement signal are representative of electrical conductivity adjusted for deviation of a temperature of the liquid from a reference temperature value, then the method need not involve carrying out such an adjustment. It becomes more accurate, because the measurement signal is more accurately dependent only on the concentration of dissolved components, rather than being dependent also on the activity coefficients, which vary with temperature.

In an embodiment, the measure of the concentration of components removable by the liquid treatment part corresponds to a measure of the concentration of components contributing to temporary hardness in water.

The method is particularly suitable for this measure. Hardness in water is due to magnesium and calcium ions. It comprises two components, namely temporary and permanent hardness. Temporary hardness or carbonate hardness (the two terms are used interchangeably herein) is caused by dissolved minerals with carbonate and hydrogen carbonate anions, whereas permanent hardness is associated with minerals comprising other anions, such as chloride. A common treatment part for removing carbonate hardness will include ion exchange material configured to exchange calcium and magnesium for hydrogen ions. The hydrogen ions react with carbonate and bicarbonate anions to form water and carbon dioxide. To prevent the water that is treated when newly regenerated ion exchange material is used from becoming too acidic, buffering agents are generally added. These may affect the measurement signal to such an extent that it cannot be used to obtain a measure of carbonate hardness. At least a correction may be required. Similarly, unbound minerals may be flushed out of the bed of water treatment material in the liquid treatment part to such an extent that a wildly varying measurement signal is obtained. With the present method, the signal can be disregarded during such an initial phase. Typically, the effects of buffering agents and unbound minerals and other fine particles will disappear after a certain volume of water has passed through the liquid treatment part, so that a second parameter corresponding to an integral of a variable representative of a rate of use of the liquid treatment part, in particular a rate of flow through the liquid treatment part, is suitable for determining when the measurement signal can be assumed to be reliable. Towards the end of the useful lifetime of the liquid treatment part, when only a relatively small fraction of the carbonate hardness is still removed, the value of the second parameter will indicate that the measurement signal has become unreliable again.

In a variant of this embodiment, adapting the determination of values of the measure in dependence on a current value of at least one second parameter includes carrying out a correction of a value determined on the basis of at least a current value of the measurement signal only if the current value of the second parameter is within a certain range.

This variant is useful for correcting for the effect of buffering agents on the measurement signal. The effect will generally last for a certain period until the buffering agent or agents is or are depleted. An effect is to extend the period within which measurement signal values can be used, instead of being simply disregarded.

In a variant, an amount of the correction is dependent on at least the current value of the second parameter.

This variant is able to take account of progressive changes in the rate at which buffering agents are added to water flowing through the liquid treatment part and on decreasing rates at which calcium and magnesium are removed as the liquid treatment medium nears exhaustion. To take account of the effects due to the release of buffering agents, the correction will take the form of the addition to an intermediate result of a value decreasing with increasing values of the second parameter. The intermediate result is calculated as if no components are released into the liquid.

In a variant, an amount of the correction is at least dependent on at least one value of the measure of the concentration of components removable by the liquid treatment calculated previously.

This variant takes account of the fact that the buffering agents will be added to the water at a larger rate when the carbonate hardness, represented by the measure based on at least one value of the measurement signal, is high. Typically, buffering will be effected by cation exchange material loaded with potassium, which is exchanged for calcium and magnesium. If the carbonate hardness is high, then more potassium will be released, so that the electrical conductivity change is smaller than would have been the case if hydrogen had been released in exchange for calcium and magnesium. If an intermediate result of the calculation is based on the assumption that only hydrogen is released, then the correction to take account of the fact that the assumption is not correct should be higher for higher levels of carbonate hardness. In one variant, the amount of the correction is further dependent on a measure of a different concentration of components on which the value of the first parameter depends. The measure of the different concentration may be calculated on the basis of both a value of the measurement signal and the at least one value of the measure of the concentration of components removable by the liquid treatment calculated previously. Thus, the correction can be dependent on the carbonate hardness and the permanent hardness, for example. This takes account of the fact that both may influence the rate of depletion of buffering agents. In an embodiment, the amount of the correction is determined iteratively: a corrected value of the measure is calculated; this measure is then used to determine a new value of the correction; the new value of the correction is used to determine a new value of the measure, etc., until a criterion for breaking off the iterations is met. This criterion may be a convergence criterion and/or a maximum number of allowed iterations.

In a further variant, an upper limit of the certain range is calculated on the basis of at least one value of the measure of the concentration of components removable by the liquid treatment part calculated previously.

This variant will generally be combined with the variant in which the amount of the correction is dependent on at least one value of the measure of the concentration of components removable by the liquid treatment calculated previously. In a variant, the upper limit is further based on at least one value of the measurement signal and a calculation of a measure of a different concentration of components on which the value of the first parameter depends. Where an ion exchange material is used to provide buffering agents, the counter ions will be exchanged for both permanent and carbonate hardness-inducing components. This variant of the method takes account of this fact. Given a fixed amount of buffering agents, a higher measure of the different concentration of components that are also removed in exchange for the buffering agents implies a smaller proportion is available for exchange against components removable by the liquid treatment part. Given a particular rate of depletion, a smaller available amount means that the phase in which the release of buffering agents needs to be taken into account will terminate after a smaller increase in the second parameter. Thus, if the second parameter corresponds, for example, to the aggregate volume of water that has been led through the liquid treatment part, then the phase in which the release of buffering agents is taken into account should be shorter (in terms of water volume).

In another embodiment of the method, a value of the measure obtainable on the basis of at least a current value of the measurement signal is provided as input for a further process only upon determining that the current value of at least one of the second parameters is above a certain minimum value.

In one variant, no current value of the measure is calculated. In another variant, it is calculated, but not provided as input for the further process. Because current values of the measurement signal are not used unless the current value of the second parameter is above a certain minimum value, the measurement signal is not used at the start of use of a newly regenerated liquid treatment medium. Effects on the measurement signal due to the flushing out of components such as unbound minerals cannot lead to the use of a correspondingly varying measure of the concentration of removable components in a further process.

In a variant, the further process is carried out independently of current values of the measurement signal for as long as the current value of at least one of the second parameters is below the certain minimum value.

The further process can be carried out on the basis of a default value of the first parameter or on the basis of a stored last reliable value of the measurement signal. It need not be interrupted just because a reliable current value of the measurement signal is temporarily unavailable.

In an embodiment of the method, a value of the measure obtainable on the basis of at least a current value of the measurement signal is provided as input for a further process only upon determining that the current value of at least one of the second parameters is below a certain maximum value.

Thus, when liquid treatment medium in the liquid treatment part is at or nearing the point of exhaustion, current values of the measurement signal are no longer used. Since the signal is a signal originating from a sensor located downstream of the liquid treatment part, the step of determining values of the measure of the concentration of components removable by the liquid treatment part on the basis of at least respective value of the measurement signal will involve the execution of an algorithm based on an assumed extent to which the liquid treatment part removes these components. Such assumptions are no longer valid near the point of exhaustion of the liquid treatment media comprised in the liquid treatment part.

In an embodiment, adapting the determination of values of the measure in dependence on a current value of at least one of the second parameters includes at least one of

adjusting a value of a variable representative of the extent to which the liquid treatment part removes the at least one component from liquid flowing through the liquid treatment part in dependence on the current value of at least one of the second parameters and

adding to an intermediate result a value depending at least on the current value of at least one of the second parameters.

This embodiment allows the extent to which the liquid treatment media of the liquid treatment part are exhausted to be taken into account. Current values of the measurement signal can thus be used to yield relatively accurate values of the measure of the concentration of components removable by the liquid treatment part over a larger fraction of the total useful lifetime of the liquid treatment media of the liquid treatment part.

Adding to an intermediate result a value depending on the current value of at least one of the second parameters is useful to take account of the release of components affecting the first parameter in the liquid treatment system upstream of the sensor, in particular in the liquid treatment part. The value added to the intermediate value may further depend on a value of the measure of the concentration of components removable by the liquid treatment part calculated previously, as set out above.

In an embodiment, at least if the current value of at least one of the second parameters is within a certain range, a current value of the measurement signal is used to provide an input to the further process only if more than a certain amount of liquid has been led through the liquid treatment part subsequent to an interruption of a flow of liquid through the liquid treatment part.

This embodiment takes account of the fact that the contact time of the liquid with the liquid treatment medium may have an influence on the first parameter, so that the first few values after flow has resumed are likely to deviate substantially from subsequent values. At least in a phase in the useful life of the liquid treatment part in which the liquid treatment medium or media have lost some effectiveness, the effect of the contact time is likely to be significant enough to warrant ignoring the first few values. This is especially true if the liquid treatment part includes some dead volume surrounding the liquid treatment medium with which the liquid is brought into contact.

In a variant, the current value of the measurement signal that is used is averaged with at least one previous value of the measurement signal, obtained upon determining that more than a certain amount of liquid had been led through the liquid treatment part subsequent to an interruption of a flow of liquid through the liquid treatment part.

This variant further tends to improve the reliability of the value of the measure of the concentration of components removable by the liquid treatment part by partially neutralising the effect of outlying measurement signal values due to stagnant liquid. It is assumed that liquid is withdrawn in bursts, e.g. corresponding to the amount of water needed for a rinsing process in a dish washer. Only the measurement signal value pertaining at the end of the withdrawal is used, provided the rinsing process uses enough water. The rinsing process may be the first one of the evening in a restaurant, so that in fact this water has remained stagnant in the liquid treatment part from the end of the previous evening to the start of the next evening's period of regular withdrawals. The measurement signal value obtained at the end of the first withdrawal of the evening may still represent an outlier, especially towards the end of the useful lifetime of the liquid treatment medium in the liquid treatment part. Averaging over e.g. five consecutive withdrawals will tend to reduce the effect of this outlier.

According to another aspect, the system for processing data according to the invention is characterised in that the system is configured to effect at least one of:

(i) a determination of whether to provide a current value of the measure obtainable on the basis of at least a current value of the measurement signal as input for a further pro-process; and
(ii) an adaptation of the determination of values of the measure

in dependence on a current value of at least one of the second parameters.

In an embodiment, the system is configured to carry out a method according to the invention.

According to another aspect, the liquid treatment system according to the invention includes a liquid treatment device containing at least a liquid treatment part including at least one liquid treatment medium for removing at least one component of liquid flowing through the liquid treatment part to at least some extent; and
a system for processing data according to the invention.

According to another aspect of the invention, there is provided a computer program, including a set of instructions capable, when incorporated in a machine-readable medium of causing a system having information processing capabilities to carry out a method according to the invention.

The invention will be explained in further detail with reference to the accompanying drawings, in which:

Fig. 1 is a schematic diagram of a water treatment apparatus including a system for monitoring and controlling temporary hardness;
Fig. 2 is a schematic diagram of a variant of the water treatment apparatus of Fig. 1 including a fluid treatment device in the form of a replaceable cartridge;
Fig. 3 is a state diagram illustrating how a blending fraction is controlled in dependence on the current phase in the useful life of the replaceable cartridge of Fig. 2;
Fig. 4 is a flow chart illustrating a method of determining a measure of the temporary hardness as executed by a data processing system associated with the apparatus of Fig. 2 during one of the phases of Fig. 3;
Fig. 5 is a diagram illustrating a correction as carried out during one of the phases of Fig. 3;
Fig. 6 is a schematic diagram of a further water treatment apparatus; and
Fig. 7 is a flow chart illustrating a variant of the method of Fig. 4 as executed by a control device associated with the apparatus of Fig. 6.

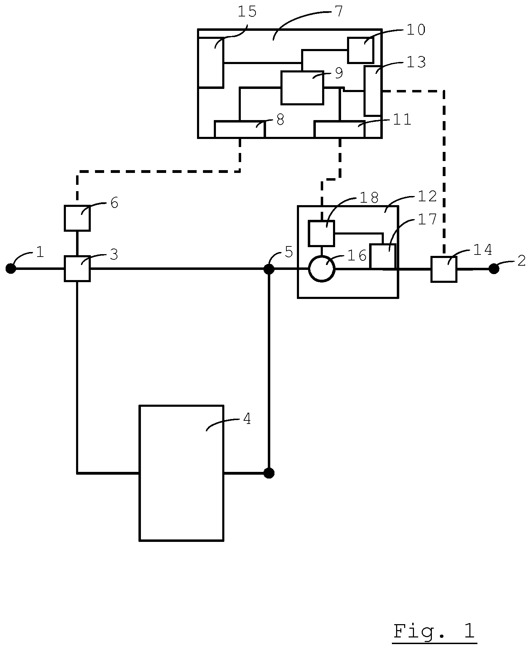

A system for softening water (Fig. 1), as an example of a liquid treatment system, includes an inlet 1 for connection to a supply of untreated water. The supply of water can be the mains water supply, for example. The system includes an outlet 2 for connection to an appliance (not shown) or a conduit leading to an appliance, in order to supply water with an appropriate hardness level to the appliance. The system may be configured to supply water to more than one appliance of course. A first and second fluid path lead through the system between the inlet 1 and the outlet 2.

The untreated water entering the system through the inlet 1 is led to a variable-ratio flow divider 3, where the first and second fluid paths go separate ways. The first fluid path passes through a liquid treatment part 4 configured to remove minerals contributing to temporary or carbonate hardness to at least a certain extent from water led through it.

The liquid treatment part 4 includes a bed of at least one liquid treatment medium. The liquid treatment medium includes at least one type of ion exchange resin, in particular a cation exchange resin in the hydrogen form. The cation exchange resin may be of the weakly acidic type. A least initially, the liquid treatment medium is effective to remove all temporary hardness from the water passing through it.

Calcium and magnesium ions are removed from the water in exchange for hydrogen ions. The hydrogen ions react with carbonate and hydrogen carbonate ions, in the latter case forming water and carbon dioxide. As a result, the total ion concentration is reduced, causing a change in the electrical conductivity of the water, as will be explained. The pH of the water is also reduced. To counter this reduction in pH, at least initially, the liquid treatment part 4 may further include buffering agents to give the treated water a stable pH value within a certain range. The lower bound of the range may be a value between 5 and 7, for example. The upper bound of the range may be a value between 7 and 9, for example.

The second fluid path bypasses the liquid treatment part 4 such that components contributing to temporary hardness and removable from the water by the liquid treatment part 4 remain in liquid led through the second fluid path to at least a certain higher extent than in liquid led through the first fluid path. In the system illustrated in Fig. 1, water led through the second fluid path is not treated at all. In other embodiments, it is treated differently, e.g. to remove pathogens or organic compounds from the water.

The first and second fluid paths join at a mixing location 5 such that the water led through the first fluid path and treated in the liquid treatment part 4 mixes with the water led through the second fluid treatment path. As a result, even though the liquid treatment part 4 is at least initially configured to remove all components contributing to temporary hardness from the water led through it, the water provided at the outlet 2 can have any degree of temporary hardness up to that of the untreated water.

The fraction of the total volume of water passing through the outlet 2 per unit of time that has been led through the second fluid path is referred to as the blending fraction. Its value is determined by the settings of the variable ratio flow divider 3.

The flow divider 3 can be adjusted to set the blending fraction by means of an electric motor 6, e.g. a stepper motor or a servo motor. The motor 6 is controlled by a control device 7.

The control device 7 includes an interface 8 to the electric motor 6, a data processing unit 9 and memory 10, an interface 11 to a sensor device 12 and an interface 13 to a flow meter 14. The control device 7 also includes a further interface 15. The further interface 15 includes at least one of a user interface and a data exchange interface, the former for receiving user input and/or providing output in a perceptible form and the latter for exchanging data with an external device, e.g. an appliance arranged to receive water provided at the outlet 2.

The further interface 15 is used in one embodiment to obtain data representative of a target value of the carbonate hardness of the water downstream of the mixing location 5. This data may be as simple as an indication of the type of application for which the water is intended, in which case the control device 7 uses stored date to determine the target value. The further interface 15 can be used to provide at least one of data representative of the actual carbonate hardness of the water downstream of the mixing location 5 and/or of the water received at the inlet 1, data indicating the level of exhaustion of the liquid treatment medium in the liquid treatment part 4 and data indicating that a certain level of exhaustion has been reached.

The sensor device 12 includes an electrical conductivity sensor 16 arranged to measure the electrical conductivity of the water downstream of the mixing location 5.

The electrical conductivity of water is dependent on the concentration of dissolved ions of all species, not just those contributing to hardness or that fraction of those species contributing to hardness that contributes to temporary hardness. Thus, for example, the water may contain dissolved calcium chloride, of which the calcium ions do not contribute to temporary hardness, but do contribute to permanent hardness. Moreover, certain ion species do not contribute to hardness at all, but their concentration partly determines the electrical conductivity of the water. The control device 7, specifically the data processing unit 9, is programmed to determine the temporary hardness of the untreated water received at the inlet 1 using measurement values obtained from the sensor device 12.

In the illustrated embodiment, the sensor device 12 includes a temperature sensor 17 and a data processor 18 for converting electrical conductivity values from the electrical conductivity sensor 16 into values that would have been obtained if the water had had a temperature at a certain reference value, e.g. 25°C. These corrected measurement values are provided to the control device 7 and are representative of a first parameter used in a method of determining the temporary hardness of untreated water arriving at the inlet 1. The correction in dependence on deviations from a reference temperature takes account of the fact that the electrical conductivity for a given concentration varies with temperature. The temperature signal need not be provided to the control device 7 saving on connectors and leads and reducing the potential for malfunctioning.

The electrical conductivity of the water at the location of the sensor device 12, i.e. downstream of the mixing location 5, is dependent on the electrical conductivity of the untreated water, the blending fraction and the electrical conductivity of the water directly downstream of the liquid treatment part 4 but upstream of the mixing location 5. Let the electrical conductivity of the untreated water be $s_0$ and the electrical conductivity of the water between the liquid treatment part 4 and the mixing location 5 be $s_1$. In the absence of any buffering, the difference $\Delta s \equiv s_0 - s_1$ is due to the removal of temporary hardness, i.e. representative of a change in electrical conductivity due to the treatment by the liquid treatment part 4. For a given blending fraction *x*, the electrical conductivity *s(x)* at the location of the sensor device 12 can be written by Equation (1), repeated here for ease of reference:

$$s(x) = x \cdot s_0 + (1-x) \cdot s_1 = (s_0 - s_1) \cdot x + s_1 = \Delta s \cdot x + s_1 \ . \qquad (1)$$

An approximation of the derivative *s'(x)* of the electrical conductivity *s* with respect to the blending fraction *x* gives a relatively good estimate of the change *Δs* in electrical conductivity due to the treatment by the liquid treatment part 4. Assuming that the liquid treatment part 4 is arranged only to remove all components contributing to temporary hardness, this value *Δs* can be directly converted into a measure of the temporary hardness of the untreated water. However in certain phases of the useful lifetime of the liquid treatment part 4, the electrical conductivity values *s(x)* may be too unreliable. In other phases, corrections may need to be applied to improve the accuracy of the measure of temporary hardness calculated in this way. The control device 7 is programmed to take account of this.

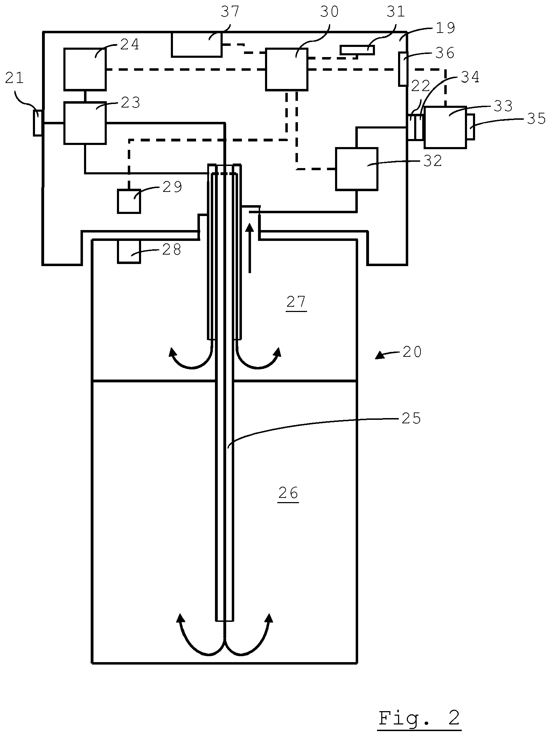

Before proceeding to a discussion of an implementation of such a phase-based method of determining a measure of the temporary hardness of the water received at the inlet and of controlling the flow divider 3 to provide water with a desired level of temporary hardness at the outlet 2, a variant of the liquid treatment system illustrated in Fig. 1 will be discussed with reference to Fig. 2.

This second liquid treatment system includes a filter head 19 and a replaceable liquid treatment cartridge 20. Mechanical interfaces on the filter head 19 and liquid treatment cartridge 20 enable the latter to be mechanically connected to the filter head 19 in an essentially fluid-tight manner.

The filter head 19 includes an inlet connector 21 for connection to a supply line for supplying untreated water. This can be mains water, for example. The filter head 19 further includes an outlet connector 22 for connection to a conduit (not shown) for delivering treated water to one or more appliances (not shown).

A first fraction of the untreated water entering the filter head 19 passes through the liquid treatment cartridge 20 along a first fluid path. A second fraction of the volume of water passes through a second fluid path. The second fraction corresponds to the blending fraction. As in the first system, a variable-ratio flow divider 23 including one or more valves is provided to split the incoming flow of water into the first and second fractions. The variable-ratio flow divider 23 is adjusted by an electric motor 24, which may be a servomotor or a stepper motor, for example. In the latter case, a sensor device (not shown) may be provided to determine the settings of the variable-ratio flow divider 23.

The first and second fluid paths both pass through the liquid treatment cartridge 20, which has two separate inlets and one outlet, each arranged to be sealingly connected to associated outlets and an inlet, respectively, of the filter head 19, when the liquid treatment cartridge 20 is mechanically connected to the filter head 19. A fall tube 25 is arranged to deliver the fraction of water led along the first fluid path to a first bed 26 of liquid treatment medium. The fraction of water led along the second fluid path is led to a second bed 27 of liquid treatment medium, arranged downstream, in use, of the first bed 26 of liquid treatment medium. The first fluid path also passes through the second bed 27 of liquid treatment medium, with the consequence that it functions as a mixing location, where the first and second fluid paths join and the respective fractions of water mix. This mix of water then leaves the liquid treatment cartridge 20 through its outlet to re-enter the filter head 19.

The first bed 26 of liquid treatment medium includes an ion exchange material, for example in the form of ion exchange resin beads, that is at least initially in the hydrogen form. The use of a replaceable liquid treatment cartridge 20 allows the use of weakly acidic ion exchange resins, which cannot easily be regenerated on-site. Instead, when the resin is exhausted, the liquid treatment cartridge 20 is returned to the supplier to allow the resin to be regenerated.

The second bed 27 of liquid treatment medium may also include an ion exchange material, even a cation exchange material, but is at least configured to remove minerals contributing to temporary hardness to a lesser extent than the medium of the first bed 26. In general, the second bed 27 will not include a cation exchange material but include other types of liquid treatment medium, in particular activated carbon. It may also include buffering agents to stabilise the pH of the treated water at a level within a desired range. Again, typically, the lower bound of the range would be between 5 and 7. The upper bound of the range would generally be between 7 and 9.

The beds 26,27 will generally include predominantly granular material in a relatively loose form. One or more meshes or grids (not shown) may be provided to prevent the particles of material from being flushed out of the liquid treatment cartridge 20. However, to maintain acceptable flow rates at common pressure differentials across the filter head 19, such retaining means cannot filter out extremely fine particles. At least on first use of a new liquid treatment cartridge 20, such fine particles may be flushed out with the treated water. It has been found that these particles have an unpredictable influence on the electrical conductivity of the treated water downstream of the liquid treatment cartridge 20. The methods of determining the temporary hardness of the untreated water to be discussed more fully below therefore take account of this phenomenon.

Furthermore, the buffering agents that may be released into the water also influence the electrical conductivity of the water downstream of the liquid treatment cartridge 20. These buffering agents are depleted before the liquid treatment medium in the first bed 26 is exhausted. As a consequence, there is a phase in the useful lifetime of the liquid treatment cartridge 20 in which electrical conductivity values obtained in measurements downstream of the liquid treatment cartridge 20 are affected by the buffering agents and a phase in which they essentially reflect the effect of the treatment in the first bed 26 only (assuming the second bed 27 does not contain any ion exchange material).

Finally, it is to be expected that the flow of water through the liquid treatment cartridge 20 is intermittent, depending on the characteristics of use of the water by appliances arranged to receive water from the liquid treatment system. Initially, the medium in the first bed 26 of liquid treatment medium will be effective to remove all ions contributing to temporary hardness from water led along the first fluid path at the rates prevailing at typical pressure differentials. As the medium becomes exhausted, the extent to which temporary hardness-inducing components are removed will decrease in a predictable way. A determination of the temporary hardness of the untreated water based on electrical conductivity measurements carried out downstream of the liquid treatment cartridge 20 as described herein can take this into account during at least a certain phase in the useful lifetime of the liquid treatment cartridge 20. It is also adapted to take account of the fact that the first quantity of water to be drawn after the flow of water has been interrupted for some time will have been softened more than the water flowing subsequently, because the liquid treatment cartridge 20 has a certain void volume in which water can stand when there is no flow through the cartridge 20. Due to the longer contact time with the liquid treatment medium, this standing water will be softened more than the water that passes straight through subsequently, at least when the medium in the first bed 26 is no longer fully in the hydrogen state.

The liquid treatment cartridge 20 is provided with a machine-readable tag 28. The filter head 19 includes a device 29 for reading information from the machine-readable tag 28 of a liquid treatment cartridge 20 mechanically connected to the filter head 19. For ease of construction, the manner of reading is contactless. The tag 28 can, for example, be an optically, electrically or electromagnetically readable device, e.g. a bar code or RFID (radio-frequency identification device) chip. In an embodiment, the device 29 and tag 28 are arranged to enable the device 29 to write data to the tag 28 as well.

Data stored on the tag include at least one of an identification of the liquid treatment cartridge 20, an identification of the type of liquid treatment medium of the first bed 26, an identification of the type of liquid treatment medium of the second bed 27, a unique identification of the liquid treatment cartridge 20 (e.g. in the form of a serial number) and data representative of a degree of use of the liquid treatment cartridge or a degree of exhaustion of the liquid treatment media. The latter can be updated by writing the data to the tag 28 during use of the liquid treatment cartridge 20.

The filter head 19 further includes a data processing unit 30 and memory 31. The data processing unit 30 is arranged to control the electric motor 24 in order to achieve a mix of water with desired characteristics. It receives data from a flow meter 32 arranged to measure at least one of the volumetric rate of flow and the accumulated volume that has passed through the filter head 19. Since it controls the blending fraction, the data processing unit 30 is able to calculate the respective volumes of water that have passed through the first and second beds 26,27 of liquid treatment medium as well.

In the system of Fig. 2, a sensor device 33 of similar construction to the sensor device 12 of the first system includes a separate housing with an inlet connector 34 for fluid-tight connection to the outlet connector 22 of the filter head 19 and an outlet connector 35 for connection to a conduit (not shown) for supplying treated water to an appliance (not shown). The sensor device 33 is arranged to provide a signal representative of the electrical conductivity of the mix of water led through the first and second fluid paths, in particular a signal comprising values representative of the electrical conductivity adjusted for deviations of the temperature of the water from a reference temperature. This signal reaches the data processing unit 30 through an interface 36. Values of the signal are representative of a first parameter for use in determining the carbonate hardness of the untreated water.

A further interface 37 includes at least one of a user interface and a data exchange interface, the former for receiving user input and/or providing output in a perceptible form and the latter for exchanging data with an external device, e.g. an appliance connected to the outlet connector 35.

The further interface 37 is used in one embodiment to obtain data representative of a target value of the temporary hardness of the water downstream of the liquid treatment cartridge 20. This data may be as simple as an indication of the type of application for which the water is intended, in which case the data processing unit 30 uses stored date to determine the target value. The further interface 37 can be used to provide at least one of data representative of the actual carbonate hardness of the water downstream of the liquid treatment cartridge 20 and/or of the untreated water, data indicating the level of exhaustion of the liquid treatment medium in the first bed 26 of the liquid treatment cartridge 20 and data indicating that a certain level of exhaustion has been reached.

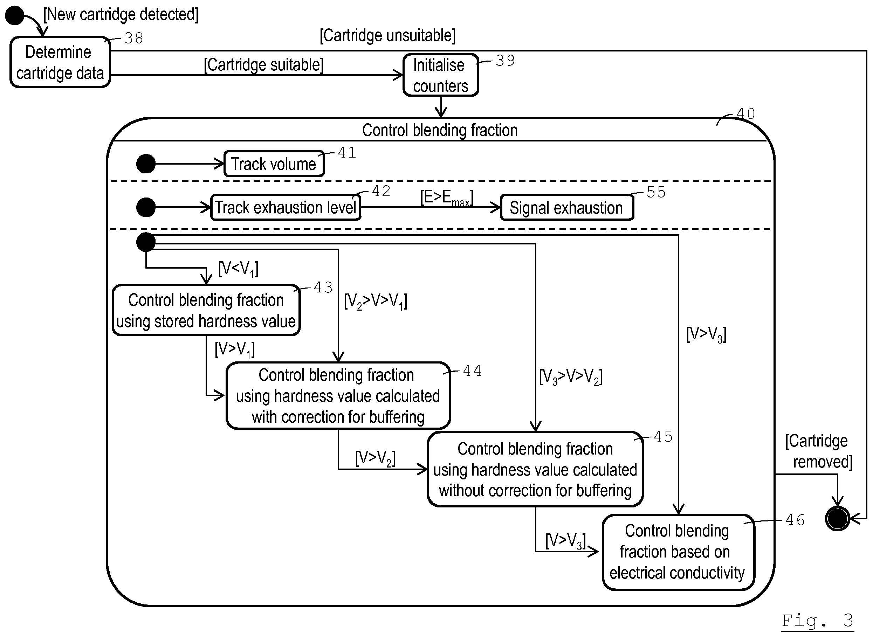

An example (Fig. 3) of a method carried out by the data processing unit 30 in order to determine the appropriate settings of the blending fraction x, and thus of the variable-ratio flow divider 23 is described from the point of detection of a new liquid treatment cartridge 20. Prior to this, the data processing unit 30 will have obtained the target value for the carbonate hardness of the water leaving the filter head 19.

Upon detecting the connection of a new liquid treatment cartridge 20, the data processing unit 30 uses (state 38) the device 29 to read data from the tag 28.

This activity includes determining the suitability of the liquid treatment cartridge 20. By way of example, a type identifier may be used to determine whether the liquid treatment media are of a type suitable to allow the method to proceed through the stages illustrated in Fig. 3. Only if the type is among a set including at least one pre-determined type does the method proceed to a next stage 39. This allows the data processing unit 30 to distinguish between liquid treatment cartridges 20 that include cation exchange medium in the hydrogen form to soften water and those that include further media to remove gypsum, for example. The latter type of liquid treatment cartridge 20 is unsuitable for the methods of determining the carbonate hardness of the untreated water using the electrical conductivity of the water downstream of the mixing location (the second bed 27 of liquid treatment medium) as outlined herein. The data processing unit 30 may use a different method (not shown) of setting the blending fraction x upon detecting a liquid treatment cartridge 20 configured to remove gypsum from the water. Similarly, if the type of cartridge 20 is unknown, the data processing unit 30 may proceed to use a method of setting the blending fraction x (not shown) that has a larger safety margin, because the extent to which carbonate hardness is reduced is not known. This would lead it to indicate a need to replace the liquid treatment cartridge 20 much sooner than the actual state of exhaustion requires. Alternatively, as illustrated, the data processing unit 30 simply returns an error message through the further interface 37 if it does not recognise the type of cartridge.

A further element of a suitability check at this stage 38 includes reading out data indicative of a state of use of the liquid treatment cartridge 20. These data are continually updated by writing current values back to the tag 28, as will be explained. Thus, if the liquid treatment cartridge 20 has been in use before being detected, the stage in its useful lifetime that it has reached can be determined. If the data indicate that the liquid treatment cartridge 20 has already reached the end of its useful lifetime, then this makes the liquid treatment cartridge 20 unsuitable. An appropriate error message is returned through the further interface 37, and the method does not proceed further.

In the example used herein, the data processing unit 30 uses second parameters in the form of first and second use measures to track the state of a liquid treatment cartridge 20.

The first use measure $V$ is simply the total volume of water that has passed through the first bed 26, i.e. the liquid treatment part in the first fluid path. In an alternative embodiment, the first use measure is representative of the total volume of water that has passed through the liquid treatment cartridge 20, i.e. the sum total of the volume of water that has been led along the first fluid path and the volume of water that has been led along the second fluid path. This alternative may be a good enough approximation of the state of use of the ex-haustible parts of the liquid treatment cartridge 20 for present purposes. It will be noted that the first use measure $V$ corresponds to an integral of a variable representative of a rate of use of the liquid treatment part (the first bed 26 of liquid treatment medium) of the liquid treatment cartridge 20 from a point of time at which liquid treatment medium in the liquid treatment part is in an initial state over all periods during which liquid flows through the liquid treatment part, this variable corresponding one-to-one with the volumetric flow rate.

A second use measure $E$ forming a second parameter is a weighted integral of the volumetric flow rate of the water that has passed through the first bed 26, i.e. the liquid treatment part in the first fluid path. The flow rate is weighted with the temporary hardness of the untreated water. Depending on the current phase of the liquid treatment cartridge 20 and on whether the filter head 19 has been used before, the value of the temporary hardness used as a weighting factor at any one particular time is a value as determined on the basis of the electrical conductivity of the water leaving the liquid treatment cartridge 20, an assumed value based on default settings or a stored value resulting from a previous determination. Again, it will be noted that the second use measure E also corresponds to an integral of a variable representative of a rate of use of the liquid treatment part (the first bed 26 of liquid treatment medium) of the liquid treatment cartridge 20 from a point of time at which liquid treatment medium in the liquid treatment part is in an initial state over all periods during which liquid flows through the liquid treatment part, this variable corresponding to the product of the current volumetric flow rate and a current value of the weighting factor.

The initial values of the use measures V,E read out on installation of the liquid treatment cartridge 20 (state 38) are used to initialise counters (state 39) in memory 31. If no values are present in the tag 28 the initial values are set to zero.

Then (composite state 40), the data processing unit 30 proceeds to an operational state, in which the blending fraction x is set by adjusting the settings of the variable-ratio flow divider 23 in a manner dependent on the current value of the first use measure $V$. To this end, the current value of the first use measure $V$ is continually updated and the current value is written to the tag 28 at regular intervals (sub-state 41).

Moreover, the current value of the second use measure E is continually updated (sub-state 42). In an embodiment, this value is also written to the tag 28 at regular intervals.

In the state 40 of controlling the blending fraction x, the data processing unit 30 is in one of four different sub-states 43-46, depending on the current value of the first use measure $V$. On the assumption that this value starts at zero (i.e. the liquid treatment cartridge 20 has not been used), a first sub-state 43 would be entered first.

During this state 43 the current value of the first parameter (the temperature-adjusted electrical conductivity) is not used to control the blending fraction $x$. In the illustrated embodiment, no current value of the temporary hardness of the untreated water is calculated at all, so that the current value of the (temperature-adjusted) electrical conductivity is ignored. Alternatively, a current value of the temporary hardness may be calculated in the same manner as in the next two sub-states 44,45, but not used as the basis for controlling the blending fraction x. Rather, if the first use measure $V$ has a value below a first value $V_1$, a value of the temporary hardness stored in memory 31 is used to set the variable-ratio flow divider 23, and thus the blending fraction $x$, to a value appropriate for achieving the target value of the temporary hardness of the mix of water provided downstream of the mixing location in the liquid treatment cartridge 20.

If available, a value of the temporary hardness calculated on the basis of the first parameter values of the water provided by the liquid treatment system when a previous liquid treatment cartridge 20 was connected is retrieved from memory 31. This value of the temporary hardness is then used in the further process of adjusting the settings of the variable-ratio flow divider 23. If the filter head 19 is being used for the first time, a value of the temporary hardness provided through the interface 37 by a user or an external device at first set-up of the filter head 19 is used. In an alternative embodiment, a factory-set default value is used. Thus, the data processing unit 30 determines whether to provide a current value of the temporary hardness obtainable on the basis of at least a current value of the (temperature-adjusted) electrical conductivity of the mix of water provided by the liquid treatment system, and the determination is made on the basis of the current value of the total volume of water that has flowed through the first bed 26 of liquid treatment medium since first use thereof. If the current value is smaller than a certain minimum value $V_1$, then the current value of the electrical conductivity (adjusted for deviations from a reference temperature) is not used. The process of controlling the blending fraction $x$ to achieve a target value of temporary hardness in water provided by the liquid treatment system is carried out independently of the current value of the (temperature-adjusted) electrical conductivity as measurable by the sensor device 33. This takes account of the fact that the electrical conductivity at first varies too much, due to the flushing out of particles present in the liquid treatment cartridge 20 and not retained by mechanical filters therein, as explained above.

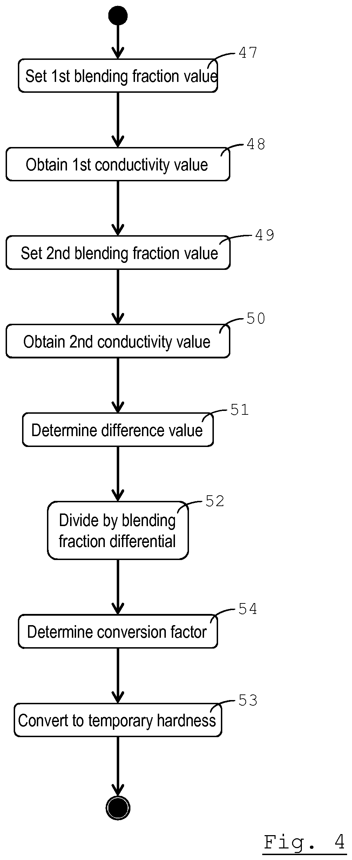

Once the value of the first use measure $V$ has exceeded the first value $V_1$, a second sub-state 44 is entered into. In this sub-state 44, a current value of the first parameter is used to calculate a current value of the carbonate hardness of the untreated water, which is then used to adjust the settings of the variable-ratio flow divider 23 in accordance with the target value for the carbonate hardness of the water downstream of the liquid treatment cartridge 20. The way in which the calculation is carried out is similar to the way in which this is done in a third sub-state 45, except that the method of the second sub-state 44 includes an extra step in which a correction to take account of the release of buffering agents is carried out. Therefore, the method of the third sub-state 45 will be explained first with reference to Fig. 4.

It will be recalled from the discussion above in relation to Equation (1) that an approximation of the derivative $s'(x)$ of the electrical conductivity s with respect to the blending fraction x gives a relatively good estimate of the change $\Delta s$ in electrical conductivity due to the treatment by the liquid treatment part 4 of the first liquid treatment system. In the second liquid treatment system, the first bed 26 of liquid treatment medium corresponds to the liquid treatment part 4 of the first liquid treatment system. Thus, an approximation of the derivative of the (temperature-adjusted) electrical conductivity s with respect to the blending fraction x is calculated in order to obtain an estimate of the change $\Delta s$ in (temperature-adjusted) electrical conductivity brought about by treatment by the liquid treatment medium in the first bed 26.

Starting from a current value $x_0$ of the blending fraction, the blending fraction is adjusted to a first value $x_1=x_0+\Delta x/2$ in a first step 47. After a delay long enough to ensure that water at the new ratio has started to reach the sensor device 33, a first value $s(x_1)$ of the first parameter (the temperature-adjusted conductivity) is obtained (step 48). Then (step 49), the blending fraction x is set to a second value $x_2=x_0-\Delta x/2$, different from the first value $x_1$. After a further delay long enough to ensure that water at the new ratio has started to reach the sensor device 33, the associated value $s(x_2)$ of the first parameter is obtained (step 50). This value being the latest available value of the first parameter, it can be regarded as the current value.

Next, the difference $s(x_1)-s(x_2)$ between the current and the preceding value of the first parameter is calculated (step 51) and divided (step 52) by the difference $\Delta x=x_1-x_2$ between the associated values of the blending fraction. The result is an estimate of the value of the change $\Delta s$ in (temperature-adjusted) electrical conductivity brought about by treatment by the liquid treatment medium in the first bed 26. On the assumption that all and only the components contributing to carbonate hardness are removed by the first bed 26 and that the water led through the second bed 27 undergoes no change in electrical conductivity, this value can be converted straightforwardly into a measure of the carbonate hardness in a further step 53 by dividing by a conversion factor F that is first determined (step 54).

A constant conversion factor F is used in one embodiment, e.g. 30 $\mu$S/°dH, where dH stands for deutsche Härte.

In another embodiment, a conversion factor F linearly dependent on at least one of the value $s(x_1)$ of the first parameter at the first value $x_1$ of the blending fraction and the value $s(x_2)$ at the second value $x_2$ of the blending fraction is used. In a particular version of this embodiment, a conversion factor F decreasing linearly with the average of the two values $s(x_1),s(x_2)$ is used, as described more fully in co-pending international patent application No. PCT/EP2013/064112, filed on 4 July 2013 by the same applicant as the present applicant. It has been found empirically and by modelling the dependency of the electrical conductivity on the mineral concentration using the Debye-Hückel-Onsager theory that such a variable conversion factor improves the accuracy of the determination of the carbonate hardness.

The change $\Delta x$ in blending fraction can be quite small, of the order of 0.2 or smaller, e.g. 0.1. As a consequence, any appliance downstream of the filter head 19 can remain connected whilst the steps 47-53 are carried out. They can be repeated relatively often, but intervals in the order of days generally suffice to capture variations in the temporary hardness of the untreated water.

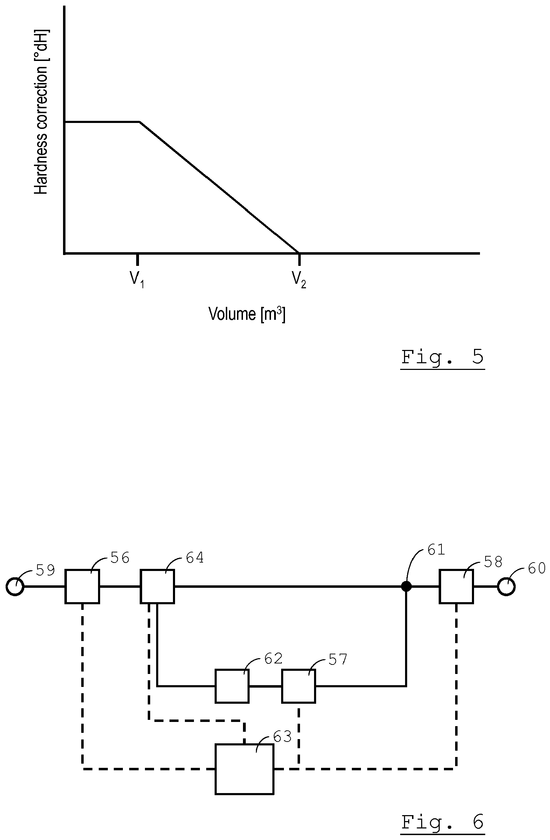

In the second sub-state 44, it is assumed that the liquid treatment part is completely effective. However, the last step 53 is followed by a further step (not shown), in which the calculated carbonate hardness is corrected by adding a value dependent on the current value of the first use measure V in a manner illustrated in Fig. 5. That is to say, the correction decreases linearly with the value of the first use measure V, reaching zero when the first use measure V has reached a second value $V_2$, which triggers the transition from the second to the third sub-state. Thus, the determination of the carbonate hardness based on at least a current value of the first parameter (the temperature-adjusted electrical conductivity) is adapted in dependence on a current value of a second parameter in the form of the first use measure V, which determines whether a correction is to be applied and partially determines what size this correction should have.

Further factors determining the size of the correction, corresponding to the height of the flat part of the curve represented in Fig. 5, are the carbonate and permanent hardness of the water itself. This is because more buffering will take place if the degree of carbonate hardness is higher, and the buffering agents are also depleted at a higher rate if the degree of permanent hardness is higher. Thus, at the start of the second sub-state 44, an iterative process is carried out. First, the carbonate hardness is calculated as if no buffering were taking place. Then, this value is corrected by a default value. In each of a number of iterations, the corrected value is used to determine a new value for the correction and the calculation of the carbonate hardness is repeated. The new value is both dependent on the carbonate hardness and on a calculated estimate of the permanent hardness. The calculated estimate is obtained on the basis of the electrical conductivity and the calculated value of the carbonate hardness, essentially by subtracting the contribution of the carbonate hardness-inducing components to the electrical conductivity from the electrical conductivity value and attributing a proportion of the remainder of the electrical conductivity value to the concentration of the hardness-inducing components. Upon reaching one of a set of at least one criterion for breaking off the iterations, the value corresponding to the height of the curve represented in Fig. 5 is fixed. The first and second values $V_1, V_2$ are then translated so as to keep the area under the curve represented in Fig. 5 at a certain value (corresponding in essence to the amount of available buffering agents at the start of the lifecycle of the cartridge 20 minus a value calculated on the basis of a measure of the permanent hardness).

In an embodiment, a further dependency is applied in the third sub-state 45, in that the conversion factor F is also dependent on the current value of a second parameter in the form of the first use measure V, or on the current value of a second parameter in the form of the second use measure E. In this way, account is taken of the fact that the liquid treatment medium in the first bed 26 is no longer able to remove all components contributing to temporary hardness at the prevailing flow rates, due to partial exhaustion of the ion exchange medium (replacement of the bound hydrogen by calcium and magnesium ions at the active sites).

When the second parameter in the form of the first use measure V has reached a certain maximum value $V_3$, the blending fraction x is no longer controlled using the calculated current value of the carbonate hardness as input. Rather, the system moves to a fourth sub-state 46, in which the blending fraction x is adjusted to achieve a particular value of the temperature-adjusted electrical conductivity. This particular value can be determined on the basis of the target value of the carbonate hardness using a conversion table, or it can be the last value prior to transition to the fourth sub-state, for example.

In the fourth sub-state 46, measurement values obtained immediately after liquid has started flowing following an interruption in the flow of liquid through the liquid treatment part (i.e. the first bed 26) are ignored. Measurement values are only used to determine an input to the further process of adjusting the blending fraction after a certain volume of liquid has been led through the liquid treatment system, more particularly the liquid treatment part. This addresses the effect caused by standing water when the ion exchange material is already appreciably exhausted as explained above.

In an embodiment, account is taken of the fact that the liquid treatment system may stand idle for a longer period of time, e.g. over the weekend when used to treat water for a company restaurant. The first volume of water withdrawn from the system at the start of the week will have a relatively low degree of carbonate hardness, because the proportion treated by the liquid treatment cartridge 20 has remained stagnant therein over the weekend. Consequently, the measurement signal will indicate a low value of the electrical conductivity. This should not lead to an immediate adjustment of the blending ratio x, because the value will increase for the next withdrawal of water. Therefore, at least one respective value obtained at the end of each of four preceding intervals corresponding to the passage of a minimum volume of water following an interruption of the flow of water are averaged with at least one obtained at the end of a latest interval corresponding to the passage of a minimum volume of water following an interruption of the flow of water. The average is used to control the blending fraction x. The interruption can be as small as 1 min. It may decrease with increasing value of the first or second use measure V,E.

All the while, the second use measure E is continually updated using the best available measure of the carbonate hardness of the untreated water (calculated in the second and third concurrent sub-states and retrieved from memory 31 in the first and fourth concurrent sub-state). On reaching a pre-determined maximum value $E_{max}$, a signal to replace the liquid treatment cartridge 20 is provided (sub-state 55). The state 40 of controlling the blending fraction is left when a user replaces the liquid treatment cartridge 20 or shuts down the system to do so. In an alternative embodiment, the system itself initiates a shut-down, e.g. upon determining that the first or second use measure *V,E* has reached a further maximum value.

The values that determine the transitions between the sub-states 43-46 and to the concurrent sub-state 55 of signalling exhaustion of the liquid treatment medium of the first bed 26 are either read from the tag 28 in the first state 38 or they are read from memory 31 using data identifying a type of the liquid treatment cartridge 20 as a key into a table of such data stored in memory 31. Thus, characteristics of the liquid treatment cartridge 20 that influence the electrical conductivity of the treated water are taken into account to achieve the most reliable input to the process of adjusting the blending fraction x to meet the target value for the carbonate hardness of the water provided by the liquid treatment system to appliances downstream thereof.

The data processing unit 9 of the first liquid treatment system executes essentially the same method as just outlined, except that it does not detect the connection of a new liquid treatment cartridge but is re-set when the liquid treatment medium in the liquid treatment part 4 is replaced or regenerated.

Instead of using only one sensor device 12,33, the method described above can be adapted for use in a third liquid treatment system (Fig. 6) that includes two sensor devices 56,57 and a flow meter 58. The third liquid treatment system as shown includes an inlet 59 for receiving untreated water and an outlet 60 for supplying a mix of water that has been led through a first fluid path and water that has been passed through a second fluid path. The first and second fluid paths join at a mixing location 61.

For simplicity, only a liquid treatment part 62 in a completely separate first fluid path is shown, and the second fluid path includes no liquid treatment medium. It will be understood that this is just a simplified representation of a system that may include the liquid treatment part 62 as a bed of liquid treatment medium in a liquid treatment cartridge through which the second fluid path also passes. The liquid treatment part 62 has the same composition as the liquid treatment part 4 of the first liquid treatment system and the first bed 26 of liquid treatment medium in the second liquid treatment system.

As in the first and second liquid treatment systems, a control device 63 controls the settings of a variable-ratio flow divider 64 that determine the blending ratio *x*.

The first sensor device 56, which corresponds in build-up to the sensor device 12 of the first liquid treatment system (Fig. 1), is situated upstream of the liquid treatment part 62. In the illustrated embodiment, it is even located upstream of the variable-ratio flow divider 64. This has the effect that it can be external to a filter head in embodiments in which the third liquid treatment system is implemented as a system comprising a filter head and a replaceable liquid treatment cartridge.

The second sensor device 57, which also corresponds in build-up to the sensor device 12 of the first liquid treatment system (Fig. 1), is located downstream of the liquid treatment part 62, but upstream of the mixing location 61.

To determine the temporary hardness of the untreated water, the method illustrated in Fig. 3 is used, but the implementation of determination of values of the carbonate hardness on the basis of at least respective values of the (temperature-adjusted) electrical conductivity measured downstream of the liquid treatment part 62 in the second and third sub-states 44,45 is different. Instead of basing a current value of the carbonate hardness on a current value of the signal from the second sensor device 57 and a preceding value at a different value of the blending ratio, it is based on a current value of that signal and a current value of the (temperature-adjusted) electrical conductivity as measured by the first sensor device 56.

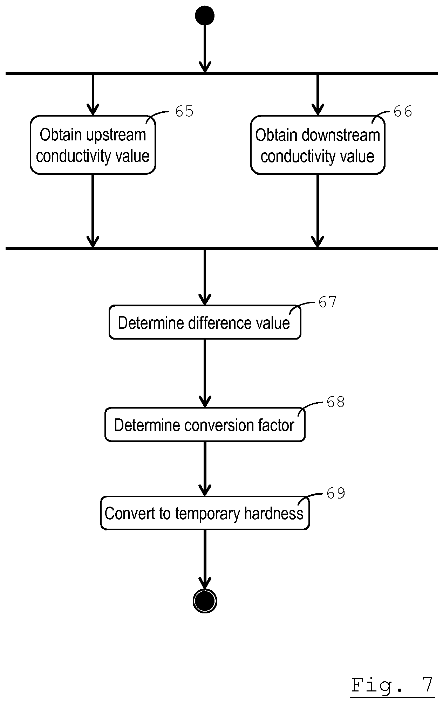

Thus, as illustrated in Fig. 7, the control device 63 obtains these two values (steps 65,66). It then determines (step 67) the difference between these two values. This is a value representative of the change in (temperature-adjusted) electrical conductivity due to the treatment of the water in the liquid treatment part 62. Thus, where the liquid treatment medium in the liquid treatment part 62 is arranged to remove essentially all components contributing to temporary hardness, the change in electrical conductivity is directly proportional to the concentration of such components in the untreated water.

The control device 63 obtains a conversion factor F in a step 68 identical to the corresponding step 54 in the method illustrated in Fig. 4. Thus, in one implementation of this step 68, a constant conversion factor F, e.g. 30 μS/°dH, is read from memory. In another implementation, the value of the factor F is determined on the basis of a linear relationship stored in memory, using e.g. an average of the two values obtained from the sensor devices 56,57. In accordance with the stored relationship, the conversion factor F decreases linearly with the absolute value of the electrical conductivity. Instead of using an average of the two electrical conductivity values, only one of the two values may be used instead, thus avoiding the need to calculate an average.

Finally (step 69), the change in temperature-adjusted electrical conductivity is divided by the value of the conversion factor *F* determined in the preceding step 68, yielding a measure of the carbonate hardness of the untreated water. This value is used as the input to a process of adjusting the settings of the variable-ratio flow divider 64 in order to achieve a target value of the carbonate hardness of the mix of treated and untreated water downstream of the mixing location 61.

The fourth sub-state 46 illustrated in Fig. 3 also requires a slight modification to be implemented in the third liquid treatment system (Fig. 6). This is because the electrical conductivity or temperature-adjusted electrical conductivity of the water downstream of the mixing location 61 is not measured directly. Instead, the value is predicted, based on the values of the untreated and treated water obtained from the sensor devices 56,57 and value of the blending fraction *x* as determined by the settings of the variable-ratio flow divider 64.

The third liquid treatment system thus also uses one or both of the variables *V,E* forming a second parameter within the meaning of the present disclosure to determine whether to provide a current value of a measure of the carbonate hardness on the basis of at least a current value of the (temperature-adjusted) electrical conductivity as measured downstream of the liquid treatment part 62 as input for a process of adjusting the settings of the variable-ratio flow divider 64. Furthermore, a current value of at least one of the variables *V,E* is used to adapt the determination of the carbonate hardness. In this way, depending on the phase in the useful lifetime of the liquid treatment medium comprised in the liquid treatment part 62, as reliable a measure as possible is used as input for the process of adjusting the settings.

The invention is not limited to the embodiments described above, which may be varied within the scope of the accompanying claims. For instance, the second and third sub-states 44,45 may be merged into one, with the correction to take account of the release of buffering agents being carried out for as long as the system is in this state. The correction of the conversion factor to take account of the gradual exhaustion of the ion exchange material may be omitted in this state, in which case the value of the second parameter (first variable *V* or second variable E forming a second parameter) at which the sub-state is left for the last sub-state 46 may be a lower one.

LIST OF REFERENCE NUMERALS

1 - Inlet

2 - Outlet

3 - Variable-ratio flow divider

4 - Liquid treatment part

5 - Mixing location

6 - Motor

7 - Control device

8 - Motor interface

9 - Data processing unit

10 - Memory

11 - Interface to sensor device

12 - Sensor device

13 - Interface to flow meter

14 - Flow meter

15 - Further interface

16 - Conductivity sensor

17 - Temperature sensor

18 - Data processor

19 - Filter head

20 - Liquid treatment cartridge

21 - Inlet connector

22 - Outlet connector

23 - Variable-ratio flow divider

24 - Motor

25 - Fall tube

26 - First bed of liquid treatment medium

27 - Second bed of liquid treatment medium

28 - Machine-readable tag

29 - Device for reading and writing data to a tag

30 - Data processing unit

31 - Memory

32 - Flow meter

33 - Sensor device

34 - Inlet connector of sensor device

35 - Outlet connector of sensor device

36 - Interface to sensor device

37 - Further interface

38 - State (determine cartridge data)

39 - State (initialise counters)

40 - Composite state (control blending fraction)

41 - Sub-state (track volume)

42 - Sub-state (track exhaustion level)

43 - Sub-state (Control blending fraction using stored hardness value)

44 - Sub-state (Control blending fraction with hard-ness value calculated with correction for buffering)

45 - Sub-state (Control blending fraction with hard-ness value calculated without correction for buffering

# EP 2 890 978 B1

46    - Sub-state (Control blending fraction based on electrical conductivity)

47    - Step (set first blending fraction value)

48    - Step (obtain first conductivity value)

49    - Step (set second blending fraction value)

50    - Step (obtain second conductivity value)

51    - Step (determine difference value)

52    - Step (divide by blending fraction differential)

53    - Step (convert to temporary hardness)

54    - Step (determine conversion factor)

55    - Sub-state (signal exhaustion)

56    - First sensor device

57    - Second sensor device

58    - Flow meter

59    - Inlet

60    - Outlet

61    - Mixing location

62    - Liquid treatment part

63    - Control device

64    - Variable-ratio flow divider

65    - Step (obtain upstream conductivity value)

66    - Step (obtain downstream conductivity value)

67    - Step (determine difference value)

68    - Step (determine conversion factor)

69    - Step (convert to temporary hardness)

## Claims

1. Method of processing data obtained by operating a liquid treatment system including a liquid treatment part (4;26;62) containing at least one liquid treatment medium, which method includes:

> receiving a measurement signal, values of which are representative of a first parameter, the first parameter being a parameter of the liquid depending partially on a concentration of at least one component removable to at least some extent by the liquid treatment part (4;26;62) from liquid flowing through the liquid treatment part (4;26;62) and also on that of other components that are not removable,

wherein the signal is a signal originating from a sensor (12;33;57) located downstream of the liquid treatment part (4; 26; 62) ;

determining values of the measure of the concentration of components removable by the liquid treatment part (4;26;62) on the basis of at least respective values of the measurement signal; and

determining values of at least one second parameter, each second parameter corresponding to an integral of a variable from a point in time at which liquid treatment medium in the liquid treatment part (4;26;62) is in an initial state over periods during which liquid flows through the liquid treatment part (4;26;62), wherein the variable is at least dependent on one of a rate at which liquid flows through the liquid treatment part (4;26;62) during use and a rate at which liquid flows through the liquid treatment system during use,

**characterised by**

at least one of:

(i) determining whether to provide a value of the measure obtainable on the basis of at least a current value of the measurement signal as input for a further process; and

(ii) adapting the determination of values of the measure

in dependence on a current value of at least one of the second parameters.

2. Method according to claim 1,
wherein the step of determining values of the measure of the concentration of components removable by the liquid treatment part (4;26;62) includes determining a difference between a value of the first parameter at a first ratio of liquid treated by the liquid treatment part (4;26;62) to liquid untreated by the liquid treatment part (4;26;62) and a value of the first parameter at a different ratio of liquid treated by the liquid treatment part (4;26;62) to liquid untreated by the liquid treatment part (4;26;62).

3. Method according to claim 2,
wherein the liquid treatment system includes:

a first fluid path passing through the liquid treatment part (4; 26);
a second fluid path bypassing the liquid treatment part (4;26), such that components removable by the liquid treatment part (4;26) remain in liquid led through the second fluid path to at least a certain higher extent than in liquid led through the first fluid path;
a mixing location (5;27), where the first and second fluid paths join to mix liquid led through the first and second fluid paths; and
a device (3;23) for adjusting a blending fraction corresponding to a proportion of liquid led through the second fluid path in liquid downstream of the mixing location (5;27),
wherein the measurement signal is a signal originating from a sensor (12;33) located downstream of the mixing location (5;27), and
wherein the values of the first parameter at the first and second ratios are obtained by causing the device (3;23) to adjust the blending fraction.

4. Method according to any one of the preceding claims,
wherein the liquid treatment system includes:

a first fluid path passing through the liquid treatment part (4; 26; 62) ;
a second fluid path bypassing the liquid treatment part (4;26;62), such that at least one component removable by the liquid treatment part (4;26;62) remains in liquid led through the second fluid path to at least a certain higher extent than in liquid led through the first fluid path;
a mixing location (5;27;61), where the first and second fluid paths join to mix liquid led through the first and second fluid paths; and
a device (3;23;64) for adjusting a blending fraction corresponding to a proportion of liquid led through the second fluid path in liquid downstream of the mixing location (5;27;61), and
wherein the further process includes causing the device (3;23;64) to adjust the blending fraction.

5. Method according to any one of the preceding claims,
wherein the measure of the concentration of components removable by the liquid treatment part (4;26;62) corresponds to a measure of the concentration of components contributing to temporary hardness in water.

**6.** Method according to claim 5,
wherein adapting the determination of values of the measure in dependence on a current value of at least one second parameter includes carrying out a correction of a value determined on the basis of at least a current value of the measurement signal only if the current value of the second parameter is within a certain range.

**7.** Method according to claim 6,
wherein an amount of the correction is dependent on at least the current value of the second parameter.

**8.** Method according to claim 6 or 7,
wherein an amount of the correction is at least dependent on at least one value of the measure of the concentration of components removable by the liquid treatment part (4;26;62) calculated previously.

**9.** Method according to any one of claims 6-8,
wherein an upper limit of the certain range is calculated on the basis of at least one value of the measure of the concentration of components removable by the liquid treatment part (4;26;62) calculated previously.

**10.** Method according to any one of the preceding claims,
wherein a value of the measure obtainable on the basis of at least a current value of the measurement signal is provided as input for a further process only upon determining that the current value of at least one of the second parameters is above a certain minimum value.

**11.** Method according to any one of the preceding claims,
wherein, at least if the current value of at least one of the second parameters is within a certain range, a current value of the measurement signal is used to provide an input to the further process only if more than a certain amount of liquid has been led through the liquid treatment part (4;26;62) subsequent to an interruption of a flow of liquid through the liquid treatment part (4;26;62).

**12.** System for processing data obtained by operating a liquid treatment system including a liquid treatment part (4;26;62) containing at least one liquid treatment medium,
wherein the system includes an interface (36) for receiving a measurement signal, values of which are representative of a first parameter, the first parameter being a parameter of the liquid depending partly on a concentration of at least one component removable to at least some extent by the liquid treatment part (4;26;62) from liquid flowing through the liquid treatment part (4;26;62) and also on that of other components that are not removable,
wherein the system is configured to process a measurement signal originating from a sensor (12;33;57) located downstream of the liquid treatment part (4;26;62),
wherein the system is configured to determine values of the measure of the concentration of components removable to at least some extent by the liquid treatment part (4;26;62) from liquid flowing through the liquid treatment part (4;26;62), and
wherein the system is configured to determine values of at least one second parameter, each second parameter corresponding to an integral of a variable from a point in time at which liquid treatment medium in the liquid treatment part (4;26;62) is in an initial state over periods during which liquid flows through the liquid treatment part (4;26;62), wherein the variable is at least dependent on one of a rate at which liquid flows through the liquid treatment part (4;26;62) during use and a rate at which liquid flows through the liquid treatment system during use,
**characterised in that**
the system is configured to effect at least one of:

(i) a determination of whether to provide a value of the measure obtainable on the basis of at least a current value of the measurement signal as input for a further process; and
(ii) an adaptation of the determination of values of the measure

in dependence on a current value of at least one of the second parameters.

**13.** System according to claim 12,
configured to carry out a method according to any one of claims 1-11.

**14.** Liquid treatment system, including:

a liquid treatment device containing at least a liquid treatment part (4;26;62) including at least one liquid treatment medium for removing at least one component of liquid flowing through the liquid treatment part (4;26;62) to at least some extent; and

a system for processing data according to claim 12 or 13.

15. Computer program, including a set of instructions capable, when incorporated in a machine-readable medium of causing a system (7;30;63) having information processing capabilities to carry out a method according to any one of claims 1-11.

**Patentansprüche**

1. Verfahren zum Verarbeiten durch Betreiben eines Flüssigkeitsbehandlungssystems, das einen Flüssigkeitsbehandlungsteil (4; 26; 62) aufweist, der mindestens ein Flüssigkeitsbehandlungsmedium enthält, erzielten Daten, welches Verfahren Folgendes umfasst:

ein Empfangen eines Messsignals, dessen Werte für einen ersten Parameter repräsentativ sind, wobei der erste Parameter ein Parameter der Flüssigkeit ist, der teilweise von einer Konzentration mindestens einer Komponente, die in mindestens einem bestimmten Ausmaß durch den Flüssigkeitsbehandlungsteil (4; 26; 62) aus der Flüssigkeit, die durch den Flüssigkeitsbehandlungsteil (4; 26; 62) fließt, entfernbar ist, und auch von der anderer, nicht entfernbarer, Komponenten abhängt,

wobei das Signal ein Signal ist, das von einem Sensor (12; 33; 57) stammt, der stromabwärts des Flüssigkeitsbehandlungsteils (4; 26; 62) liegt;

ein Bestimmen von Werten des Maßes der Konzentration von Komponenten, die durch den Flüssigkeitsbehandlungsteil (4; 46; 62) entfernbar sind, basierend auf mindestens jeweiligen Werten des Messsignals, und

ein Bestimmen von Werten mindestens eines zweiten Parameters, wobei jeder zweite Parameter einem Integral einer Variablen von einem Zeitpunkt, zu dem sich das Flüssigkeitsbehandlungsmedium in dem Flüssigkeitsbehandlungsteil (4; 26; 62) in einem anfänglichen Zustand befindet, über Perioden, während welcher Flüssigkeit durch den Flüssigkeitsbehandlungsteil (4; 26; 62) fließt, entspricht, wobei die Variable zumindest von einer Rate, mit der Flüssigkeit durch den Flüssigkeitsbehandlungsteil (4; 26; 62) während des Gebrauchs fließt, oder einer Rate, mit der Flüssigkeit durch das Flüssigkeitsbehandlungssystem während des Gebrauchs fließt, abhängig ist,

**gekennzeichnet durch**:

(i) ein Bestimmen, ob ein Wert des Maßes, der auf der Basis mindestens eines aktuellen Werts des Messsignals erzielbar ist, als Eingabe für einen weiteren Prozess bereitgestellt werden soll, und/oder

(ii) ein Anpassen der Bestimmung von Werten des Maßes

in Abhängigkeit von einem aktuellen Wert mindestens eines der zweiten Parameter.

2. Verfahren nach Anspruch 1,

wobei der Schritt des Bestimmens von Werten der Messung der Konzentration von Komponenten, die durch den Flüssigkeitsbehandlungsteil (4; 26; 62) entfernbar sind, das Bestimmen eines Unterschieds zwischen einem Wert des ersten Parameters bei einem ersten Verhältnis von Flüssigkeit, die durch den Flüssigkeitsbehandlungsteil (4; 26; 62) behandelt wird, zu Flüssigkeit, die nicht von dem Flüssigkeitsbehandlungsteil (4; 26; 62) behandelt wird, und einem Wert des ersten Parameters bei einem unterschiedlichen Verhältnis von Flüssigkeit, die durch den Flüssigkeitsbehandlungsteil (4; 26; 62) behandelt wird, zu Flüssigkeit, die nicht von dem Flüssigkeitsbehandlungsteil (4; 26; 62) behandelt wird, aufweist.

3. Verfahren nach Anspruch 2,

wobei das Flüssigkeitsbehandlungssystem umfasst:

einen ersten Fluidweg, der durch den Flüssigkeitsbehandlungsteil (4; 26) durchgeht;

einen zweiten Fluidweg, der den Flüssigkeitsbehandlungsteil (4; 26) derart umgeht, dass Komponenten, die durch den Flüssigkeitsbehandlungsteil (4; 26) entfernbar sind, in Flüssigkeit, die durch den zweiten Fluidweg geführt wird, zu mindestens einem bestimmten höheren Ausmaß verbleiben als in Flüssigkeit, die durch den ersten Fluidweg geführt wird;

eine Mischstelle (5; 27), an der sich der erste und der zweite Fluidweg vereinen, um Flüssigkeit zu mischen, die durch den ersten und den zweiten Fluidweg geführt wurde, und

eine Vorrichtung (3; 23) zum Einstellen eines Verschnittanteils, der einer Proportion von Flüssigkeit entspricht, die durch den zweiten Fluidweg in Flüssigkeit stromabwärts der Mischstelle (5; 27) geführt wird,

wobei das Messsignal ein Signal ist, das von einem Sensor (12; 33) stammt, der stromabwärts der Mischstelle (5; 27) liegt, und

wobei die Werte des ersten Parameters mit dem ersten und dem zweiten Verhältnis erzielt werden, indem die Vorrichtung (3; 23) veranlasst wird, den Verschnittanteil anzupassen.

4. Verfahren nach einem der vorhergehenden Ansprüche,

wobei das Flüssigkeitsbehandlungssystem aufweist:

einen ersten Fluidweg, der durch den Flüssigkeitsbehandlungsteil (4; 26; 62) durchgeht;
einen zweiten Fluidweg, der den Flüssigkeitsbehandlungsteil (4; 26; 62) derart umgeht, dass mindestens eine Komponente, die durch den Flüssigkeitsbehandlungsteil (4; 26; 62) entfernbar ist, in Flüssigkeit, die durch den zweiten Fluidweg geführt wird, zu mindestens einem bestimmten höheren Ausmaß verbleibt als in Flüssigkeit, die durch den ersten Fluidweg geführt wird;
eine Mischstelle (5; 27; 61), an der sich der erste und der zweite Fluidweg vereinen, um Flüssigkeit zu mischen, die durch den ersten und den zweiten Fluidweg geführt wurde, und
eine Vorrichtung (3; 23; 64) zum Einstellen eines Verschnittanteils, der einer Proportion von Flüssigkeit entspricht, die durch den zweiten Fluidweg in Flüssigkeit stromabwärts der Mischstelle (5; 27; 61) geführt wird, und

wobei der weitere Prozess ein Bewirken, dass die Vorrichtung (3; 23; 64) den Verschnittanteil anpasst, umfasst.

5. Verfahren nach einem der vorhergehenden Ansprüche,

wobei das Maß der Konzentration von durch den Flüssigkeitsbehandlungsteil (4; 26; 62) entfernbaren Komponenten, einem Maß der Konzentration von Komponenten entspricht, die zu Karbonhärte in Wasser beitragen.

6. Verfahren nach Anspruch 5,

wobei das Anpassen der Bestimmung von Werten des Maßes in Abhängigkeit von einem aktuellen Wert mindestens eines zweiten Parameters ein nur, wenn der aktuelle Wert des zweiten Parameters innerhalb eines bestimmten Bereichs liegt, Durchführen einer Korrektur eines auf der Basis mindestens eines aktuellen Werts des Messsignals bestimmten Werts, umfasst.

7. Verfahren nach Anspruch 6,

wobei ein Umfang der Korrektur von mindestens dem aktuellen Wert des zweiten Parameters abhängt.

8. Verfahren nach Anspruch 6 oder 7,

wobei ein Umfang der Korrektur zumindest von wenigstens einem Wert des Maßes der Konzentration von durch den Flüssigkeitsbehandlungsteil (4; 26; 62) entfernbaren Komponenten, der zuvor berechnet wurde, abhängt.

9. Verfahren nach einem der Ansprüche 6 bis 8,

wobei ein oberes Limit des bestimmten Bereichs auf der Basis mindestens eines Werts des Maßes der Konzentration von durch den Flüssigkeitsbehandlungsteil (4; 26; 62) entfernbaren Komponenten, der vorab berechnet wurde, berechnet wird.

10. Verfahren nach einem der vorhergehenden Ansprüche,

wobei ein Wert des Maßes, der auf der Basis mindestens eines aktuellen Werts des Messsignals erzielbar ist, nur nach Feststellung, dass der aktuelle Wert wenigstens eines der zweiten Parameter oberhalb eines bestimmten Mindestwerts liegt, als Eingabe für einen weiteren Prozess bereitgestellt wird.

11. Verfahren nach einem der vorhergehenden Ansprüche,

wobei, zumindest wenn der aktuelle Wert wenigstens eines der zweiten Parameter innerhalb eines bestimmten Bereichs liegt, ein aktueller Wert des Messsignals nur, wenn im Anschluss an eine Unterbrechung des Flüssigkeitsstroms durch den Flüssigkeitsbehandlungsteil (4;26;62) mehr als eine bestimmte Flüssigkeitsmenge durch den Flüssigkeitsbehandlungsteil (4;26;62) geführt wurde, zum Liefern einer Eingabe an einem weiteren Prozess verwendet wird.

12. System zum Verarbeiten von Daten, die durch Betreiben eines Flüssigkeitsbehandlungssystems erzielt werden, das einen Flüssigkeitsbehandlungsteil (4; 26; 62) aufweist, der mindestens ein Flüssigkeitbehandlungsmedium enthält,

wobei das System eine Schnittstelle (36) zum Empfangen eines Messsignals aufweist, dessen Werte für einen ersten Parameter repräsentativ sind, wobei der erste Parameter ein Parameter der Flüssigkeit ist, der teilweise von einer Konzentration mindestens einer Komponente, die in mindestens einem bestimmten Ausmaß durch den Flüssigkeitsbehandlungsteil (4; 26; 62) aus der Flüssigkeit, die durch den Flüssigkeitsbehandlungsteil (4; 26; 62) fließt, entfernbar ist, und auch von der anderer, nicht entfernbarer, Komponenten abhängt,
wobei das System konfiguriert ist, um ein Messsignal zu verarbeiten, das von einem Sensor (12; 33; 57) stammt, der stromabwärts des Flüssigkeitsbehandlungsteils (4; 26; 62) liegt,
wobei das System konfiguriert ist, um Werte des Maßes der Konzentration von Komponenten, die in mindestens einem bestimmten Ausmaß durch den Flüssigkeitsbehandlungsteil (4; 26; 62) aus Flüssigkeit, die durch den Flüssigkeitsbehandlungsteil (4; 26; 62) fließt, entfernbar sind, zu bestimmen, und
wobei das System konfiguriert ist, um Werte mindestens eines zweiten Parameters zu bestimmen, wobei jeder zweite Parameter einem Integral einer Variablen von einem Zeitpunkt, zu dem sich das Flüssigkeitsbehandlungsmedium in dem Flüssigkeitsbehandlungsteil (4; 26; 62) in einem anfänglichen Zustand befindet, über Perioden, während welcher Flüssigkeit durch den Flüssigkeitsbehandlungsteil (4; 26; 62) fließt, entspricht, wobei die Variable zumindest von einer Rate, mit der Flüssigkeit durch den Flüssigkeitsbehandlungsteil (4; 26; 62) während des Gebrauchs fließt, oder einer Rate, mit der Flüssigkeit durch das Flüssigkeitsbehandlungssystem während des Gebrauchs fließt, abhängig ist,
**dadurch gekennzeichnet, dass**
das System konfiguriert ist, um Folgendes auszuführen:

(i) eine Bestimmung, ob ein Wert des Maßes, der auf der Basis mindestens eines aktuellen Werts des Messsignals erzielbar ist, als Eingabe für einen weiteren Prozess bereitgestellt werden soll, und/oder
(ii) eine Anpassung der Bestimmung von Werten des Maßes

in Abhängigkeit von einem aktuellen Wert mindestens eines der zweiten Parameter.

13. System nach Anspruch 12,

das konfiguriert ist, um ein Verfahren nach einem der Ansprüche 1 - 11 auszuführen.

14. Flüssigkeitsbehandlungssystem, aufweisend:

eine Flüssigkeitsbehandlungsvorrichtung, die mindestens einen Flüssigkeitsbehandlungsteil (4; 26; 62) enthält, der mindestens ein Flüssigkeitsbehandlungsmedium zum zumindest in einem bestimmten Ausmaß Entfernen mindestens einer Komponente aus Flüssigkeit, die durch den Flüssigkeitsbehandlungsteil (4; 26; 62) fließt, aufweist, und
ein System zum Verarbeiten von Daten nach Anspruch 12 oder 13.

15. Computerprogramm, das einen Satz von Anweisungen aufweist, die, wenn in ein maschinenlesbares Medium eingegliedert, dazu im Stande sind, ein System (7; 30; 63) mit Datenverarbeitungsfähigkeiten ein Verfahren nach einem der Ansprüche 1 bis 11 ausführen zu lassen.

**Revendications**

1. Procédé de traitement de données obtenues par exploitation d'un système de traitement de liquide incluant une partie de traitement de liquide (4 ; 26 ; 62) contenant au moins un milieu de traitement de liquide, lequel procédé inclut :

la réception d'un signal de mesure, dont des valeurs sont représentatives d'un premier paramètre, le premier paramètre étant un paramètre du liquide dépendant partiellement d'une concentration d'au moins un composant éliminable au moins dans une certaine mesure par la partie de traitement de liquide (4 ; 26 ; 62) du liquide s'écoulant à travers la partie de traitement de liquide (4 ; 26 ; 62) et également de celle d'autres composants qui ne sont pas éliminables,
dans lequel le signal est un signal tirant son origine d'un capteur (12 ; 33 ; 57) situé en aval de la partie de traitement de liquide (4 ; 26 ; 62) ;
la détermination de valeurs de la mesure de la concentration de composants éliminables par la partie de traitement de liquide (4 ; 26 ; 62) sur la base d'au moins des valeurs respectives du signal de mesure ; et
la détermination de valeurs d'au moins un second paramètre, chaque second paramètre correspondant à une intégrale d'une variable depuis un instant auquel le milieu de traitement de liquide dans la partie de traitement de liquide (4 ; 26 ; 62) est dans un état initial sur des périodes pendant lesquelles un liquide s'écoule à travers la partie de traitement de liquide (4 ; 26 ; 62), dans lequel la variable est au moins dépendante de l'une d'une vitesse à laquelle le liquide s'écoule à travers la partie de traitement de liquide (4 ; 26 ; 62) pendant une utilisation et d'une vitesse à laquelle le liquide s'écoule à travers le système de traitement de liquide pendant une utilisation, **caractérisé par**
au moins l'un parmi :

(i) le fait de déterminer s'il faut fournir une valeur de la mesure pouvant être obtenue sur la base d'au moins une valeur en cours du signal de mesure en tant qu'entrée pour un processus supplémentaire ; et
(ii) l'adaptation de la détermination de valeurs de la mesure

en dépendance d'une valeur en cours d'au moins l'un des seconds paramètres.

2. Procédé selon la revendication 1,

dans lequel l'étape de détermination de valeurs de la mesure de la concentration de composants éliminables par la partie de traitement de liquide (4 ; 26 ; 62) inclut la détermination d'une différence entre une valeur du premier paramètre à un premier rapport de liquide traité par la partie de traitement de liquide (4 ; 26 ; 62) sur le liquide non traité par la partie de traitement de liquide (4 ; 26 ; 62) et une valeur du premier paramètre à un rapport différent de liquide traité par la partie de traitement de liquide (4 ; 26 ; 62) sur le liquide non traité par la partie de traitement de liquide (4 ; 26 ; 62).

3. Procédé selon la revendication 2,

dans lequel le système de traitement de liquide inclut :

un premier chemin de fluide passant par la partie de traitement de liquide (4 ; 26) ;
un second chemin de fluide contournant la partie de traitement de liquide (4 ; 26), de telle sorte que des composants éliminables par la partie de traitement de liquide (4 ; 26) restent dans le liquide conduit à travers le second chemin de fluide au moins dans une certaine mesure plus élevée que dans le liquide conduit à travers le premier chemin de fluide ;
un emplacement de mélange (5 ; 27), où les premier et second chemins de fluide se rejoignent pour mélanger le liquide conduit à travers les premier et second chemins de fluide ; et
un dispositif (3 ; 23) pour ajuster une fraction de mélange correspondant à une proportion de liquide conduit à travers le second chemin de fluide dans le liquide en aval de l'emplacement de mélange (5 ; 27),

dans lequel le signal de mesure est un signal tirant son origine d'un capteur (12 ; 33) situé en aval de l'emplacement de mélange (5 ; 27), et
dans lequel les valeurs du premier paramètre aux premier et second rapports sont obtenues en amenant le dispositif (3 ; 23) à ajuster la fraction de mélange.

4. Procédé selon l'une quelconque des revendications précédentes,

dans lequel le système de traitement de liquide inclut :

un premier chemin de fluide passant par la partie de traitement de liquide (4 ; 26 ; 62) ;
un second chemin de fluide contournant la partie de traitement de liquide (4 ; 26 ; 62), de telle sorte qu'au moins un composant éliminable par la partie de traitement de liquide (4 ; 26 ; 62) reste dans le liquide conduit à travers le second chemin de fluide au moins dans une certaine mesure plus élevée que dans le liquide conduit à travers le premier chemin de fluide ;
un emplacement de mélange (5 ; 27 ; 61), où les premier et second chemins de fluide se rejoignent pour mélanger le liquide conduit à travers les premier et second chemins de fluide ; et
un dispositif (3 ; 23 ; 64) pour ajuster une fraction de mélange correspondant à une proportion de liquide conduit à travers le second chemin de fluide dans le liquide en aval de l'emplacement de mélange (5 ; 27 ; 61), et

dans lequel le processus supplémentaire inclut le fait d'amener le dispositif (3 ; 23 ; 64) à ajuster la fraction de mélange.

5. Procédé selon l'une quelconque des revendications précédentes,

dans lequel la mesure de la concentration de composants éliminables par la partie de traitement de liquide (4 ; 26 ; 62) correspond à une mesure de la concentration de composants contribuant à une dureté temporaire de l'eau.

6. Procédé selon la revendication 5,

dans lequel l'adaptation de la détermination de valeurs de la mesure en dépendance d'une valeur en cours d'au moins un second paramètre inclut la réalisation d'une correction d'une valeur déterminée sur la base d'au moins une valeur en cours du signal de mesure seulement si la valeur en cours du second paramètre est dans une certaine plage.

7. Procédé selon la revendication 6,

dans lequel une quantité de la correction est dépendante d'au moins la valeur en cours du second paramètre.

8. Procédé selon la revendication 6 ou 7,

dans lequel une quantité de la correction est au moins dépendante d'au moins une valeur de la mesure de la concentration de composants éliminables par la partie de traitement de liquide (4 ; 26 ; 62) calculée précédemment.

9. Procédé selon l'une quelconque des revendications 6 à 8,

dans lequel une limite supérieure de la certaine plage est calculée sur la base d'au moins une valeur de la mesure de la concentration de composants éliminables par la partie de traitement de liquide (4 ; 26 ; 62) calculée précédemment.

10. Procédé selon l'une quelconque des revendications précédentes,

dans lequel une valeur de la mesure pouvant être obtenue sur la base d'au moins une valeur en cours du signal de mesure est fournie comme une entrée pour un processus supplémentaire seulement lorsqu'il est déterminé que la valeur en cours d'au moins l'un des seconds paramètres est au-dessus d'une certaine valeur minimale.

11. Procédé selon l'une quelconque des revendications précédentes,

dans lequel, au moins si la valeur en cours d'au moins l'un des seconds paramètres est dans une certaine plage, une valeur en cours du signal de mesure est utilisée pour fournir une entrée au processus supplémentaire seulement si plus d'une certaine quantité de liquide a été conduite à travers la partie de traitement de liquide (4 ; 26 ; 62) après une interruption d'un écoulement de liquide à travers la partie de traitement de liquide (4 ; 26 ; 62).

**12.** Système de traitement de données obtenues par exploitation d'un système de traitement de liquide incluant une partie de traitement de liquide (4 ; 26 ; 62) contenant au moins un milieu de traitement de liquide,

dans lequel le système inclut une interface (36) pour recevoir un signal de mesure, dont des valeurs sont représentatives d'un premier paramètre, le premier paramètre étant un paramètre du liquide dépendant en partie d'une concentration d'au moins un composant éliminable au moins dans une certaine mesure par la partie de traitement de liquide (4 ; 26 ; 62) d'un liquide s'écoulant à travers la partie de traitement de liquide (4 ; 26 ; 62) et également de celle d'autres composants qui ne sont pas éliminables,
dans lequel le système est configuré pour traiter un signal de mesure tirant son origine d'un capteur (12 ; 33 ; 57) situé en aval de la partie de traitement de liquide (4 ; 26 ; 62),
dans lequel le système est configuré pour déterminer des valeurs de la mesure de la concentration de composants éliminables au moins dans une certaine mesure par la partie de traitement de liquide (4 ; 26 ; 62) du liquide s'écoulant à travers la partie de traitement de liquide (4 ; 26 ; 62), et
dans lequel le système est configuré pour déterminer des valeurs d'au moins un second paramètre, chaque second paramètre correspondant à une intégrale d'une variable depuis un instant auquel un milieu de traitement de liquide dans la partie de traitement de liquide (4 ; 26 ; 62) est dans un état initial sur des périodes pendant lesquelles un liquide s'écoule à travers la partie de traitement de liquide (4 ; 26 ; 62), dans lequel la variable est au moins dépendante de l'une d'une vitesse à laquelle le liquide s'écoule à travers la partie de traitement de liquide (4 ; 26 ; 62) pendant une utilisation et d'une vitesse à laquelle le liquide s'écoule à travers le système de traitement de liquide pendant une utilisation,
**caractérisé en ce que**
le système est configuré pour effectuer au moins l'un parmi :

(i) le fait de déterminer s'il faut fournir une valeur de la mesure pouvant être obtenue sur la base d'au moins une valeur en cours du signal de mesure en tant qu'entrée pour un processus supplémentaire ; et
(ii) une adaptation de la détermination de valeurs de la mesure

en dépendance d'une valeur en cours d'au moins l'un des seconds paramètres.

**13.** Système selon la revendication 12,

configuré pour réaliser un procédé selon l'une quelconque des revendications 1 à 11.

**14.** Système de traitement de liquide, incluant :

un dispositif de traitement de liquide contenant au moins une partie de traitement de liquide (4 ; 26 ; 62) incluant au moins un milieu de traitement de liquide pour éliminer au moins un composant de liquide s'écoulant à travers la partie de traitement de liquide (4 ; 26 ; 62) au moins dans une certaine mesure ; et
un système de traitement de données selon la revendication 12 ou 13.

**15.** Programme d'ordinateur, incluant un jeu d'instructions capables, lorsqu'elles sont incorporées dans un support lisible par machine, d'amener un système (7 ; 30 ; 63) ayant des capacités de traitement d'informations à réaliser un procédé selon l'une quelconque des revendications 1 à 11.

Fig. 1

Fig. 2

[New cartridge detected]

Determine cartridge data — 38

[Cartridge suitable]

[Cartridge unsuitable]

Initialise counters — 39

Control blending fraction — 40

Track volume — 41

Track exhaustion level — 42 $[E>E_{max}]$ Signal exhaustion — 55

$[V<V_1]$

Control blending fraction using stored hardness value — 43

$[V_2>V>V_1]$

$[V>V_1]$

Control blending fraction using hardness value calculated with correction for buffering — 44

$[V_3>V>V_2]$

$[V>V_2]$

Control blending fraction using hardness value calculated without correction for buffering — 45

$[V>V_3]$

$[V>V_3]$

Control blending fraction based on electrical conductivity — 46

[Cartridge removed]

Fig. 3

Fig. 4

Fig. 5

Fig. 6

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102005005039 A1
- EP 2228129 A1
- WO 2010025697 A1
- EP 2013064112 W